# EUROPEAN PATENT APPLICATION

(11) **EP 4 792 795 A1**
(43) Date of publication of application: **19.08.2026**
(21) Application number: 25767750.0
(22) Date of filing: 28.02.2025
(51) Int. Cl.: A61F 13/551, A61F 13/49, A61F 13/475, A61F 13/494

(54) **ABSORBENT ARTICLE**

(30) Priority: 04.03.2024 JP 2024032576
(71) Applicant: Unicharm Corporation, Ehime 799-0111 (JP)
(72) Inventor: MATSUDA, Haruna, Kanonji-shi, Kagawa 769-1602 (JP); MIYAMA, Takuya, Kanonji-shi, Kagawa 769-1602 (JP); SHINOHARA, Yukiko, Kanonji-shi, Kagawa 769-1602 (JP)
(74) Representative: Dolleymores
(86) International application number: PCT/JP2025/007167
(87) International publication number: WO 2025/187560

(57) **Abstract**

Provided is an absorbent article (1) that has a front-rear direction, a width direction, and a thickness direction which are orthogonal to each other in an unfolded state and that has an absorbent core, wherein the absorbent article (1) has neither a fastening tape nor a post-processing tape that is used at the time of disposal of the absorbent article (1) and has an opening configuration means (M) for configuring an opening at the time of disposal of the absorbent article (1). The opening is for inserting at least a portion of the absorbent core (11) at the time of disposal, and the opening configuration means (M) is provided at a position that does not overlap the absorbent core (11) when viewed in the thickness direction.

## Description

### FIELD

The present invention relates to an absorbent article.

### BACKGROUND

As absorbent articles, disposable diapers and absorbent pads are known. An absorbent article that has absorbed excrement after use is preferably disposed of in a state in which the absorbent article has been rolled or folded with the skin-side surface thereof facing inward, so as to prevent contact with the excrement or leakage thereof. For example, Patent Documents 1 and 2 disclose an absorbent article provided with a tape (tape member, winding tape) for maintaining a rolled (wound) state at the time of disposal.

### [CITATION LIST]

### [patent document ]

[Patent Document 1] WO2018-116636
[Patent Document 2] Japanese Patent Application Publication No.2020-174961

### SUMMARY

### [TECHNICAL PROBLEM]

In recent years, in order to achieve the Sustainable Development Goals (SDGs), efficient use of resources, reduction in waste, and the like have been demanded in industry. In the absorbent articles described in Patent Documents 1 and 2, a tape for maintaining the rolled (wound) state of the absorbent article is not directly related to absorption of excrement, which is a purpose of an absorbent article, but is a member used for disposal. In other words, provision of an additional member (tape) used for disposal results in an increase in waste.

Thus, the present invention has been made in view of such an issue as described above, and is directed to provision of an absorbent article that can reduce the amount of resources used and facilitate prevention of an increase in waste, while maintaining the absorbent article in a rolled shape when the absorbent article is disposed of.

### [SOLUTION TO PROBLEM]

A main aspect of the present invention to achieve the above objective is an absorbent article having a front-back direction, a width direction, and a thickness direction orthogonal to each other in an unfolded state, the absorbent article including: an absorbent core; and an opening forming means, in a state where the absorbent article has been taken out from a container material or a state where the absorbent article has been removed from the container material, the absorbent article including neither a fastening tape nor a post-handling tape, the post-handling tape being used when the absorbent article is disposed of, the opening forming means being configured to form an opening when the absorbent article is disposed of, the opening being configured to allow insertion of at least a portion of the absorbent core when the absorbent article is disposed of, and when viewed in the thickness direction, the opening-forming means being provided at a position not overlapping the absorbent core. Other features of the present invention will become apparent from the description in the present specification and the accompanying drawings.

### [ADVANTAGEOUS EFFECT OF THE INVENTION]

According to the present invention, it is possible to provide an absorbent article that can reduce the amount of resources used and facilitate prevention of an increase in waste, while maintaining the absorbent article in a rolled shape when the absorbent article is disposed of.

### BRIEF DESCRIPTION OF DRAWINGS

FIG. 1 is a plan view of an absorbent pad 1 in an unfolded and stretched state when viewed from the skin side.
FIG. 2 is a plan view of an absorbent pad 1 in an unfolded and stretched state when viewed from the non-skin side.
FIG. 3 is a schematic cross-sectional view taken along line A-A in FIG. 1.
FIG. 4 is a diagram illustrating a procedure for bringing the pad 1 into a disposal state.
FIG. 5 is a diagram illustrating the procedure for bringing the pad 1 into the disposal state.
FIG. 6 is a diagram illustrating the procedure for bringing the pad 1 into the disposal state.
FIG. 7 is a diagram illustrating a variation of the pad 1 of a first embodiment.
FIG. 8 is a plan view of a pad 20 in an unfolded and stretched state when viewed from the skin side.
FIG. 9 is a diagram illustrating a variation of the pad 20 of a second embodiment.
FIG. 10 is a plan view of a pad 30 in an unfolded and stretched state when viewed from the skin side.
FIG. 11 is a diagram illustrating a procedure for bringing a pad 30 into the disposal state.
FIG. 12 is a diagram illustrating a procedure for bringing the pad 30 into the disposal state.

### DESCRIPTION OF EMBODIMENTS

At least following matters will become apparent from the descriptions of the present specification and the accompanying drawings.

### (Aspect 1)

An absorbent article having a front-back direction, a width direction, and a thickness direction orthogonal to each other in an unfolded state, the absorbent article including: an absorbent core; and an opening forming means, in a state where the absorbent article has been taken out from a container material or a state where the absorbent article has been removed from the container material, the absorbent article including neither a fastening tape nor a post-handling tape, the post-handling tape being used when the absorbent article is disposed of, the opening forming means being configured to form an opening when the absorbent article is disposed of, the opening being configured to allow insertion of at least a portion of the absorbent core when the absorbent article is disposed of, and when viewed in the thickness direction, the opening-forming means being provided at a position not overlapping the absorbent core.

According to Aspect 1, by using the opening-forming means when the absorbent article is disposed of, it becomes easy to maintain the rolled shape of the absorbent article without providing a post-processing tape for disposing of the absorbent article, thereby reducing the amount of resources used, and facilitating prevention of an increase in waste.

### (Aspect 2)

An absorbent article having a front-back direction, a width direction, and a thickness direction orthogonal to each other in an unfolded state, the absorbent article including: an absorbent core; and an opening forming means, in a state where the absorbent article has been taken out from a container material or a state where the absorbent article has been removed from the container material, the absorbent article not including a material having 60 g/m² or more, the opening-forming means being configured to form an opening when the absorbent article is disposed of, the opening being configured to allow insertion of at least a portion of the absorbent core when the absorbent article is disposed of, when viewed in the thickness direction, the opening-forming means being provided at a position not overlapping the absorbent core.

According to Aspect 2, by not including a material having 60 g/m² or more, the flexibility of the absorbent article is maintained as compared with the case of including the material having 60 g/m² or more, and by using the opening-forming means when the absorbent article is disposed of, the rolled shape of the absorbent article can be easily maintained, thereby reducing the amount of resources used and facilitating prevention of an increase in waste.

### (Aspect 3)

An absorbent article having a front-back direction, a width direction, and a thickness direction orthogonal to each other in an unfolded state, the absorbent article including: an absorbent core; a fixing means; and an opening forming means, in a state where the absorbent article has been taken out from a container material or a state where the absorbent article has been removed from the container material, the absorbent article not including a post-handling tape used when the absorbent article is disposed of, the fixing means being located at a non-skin-side surface of the absorbent article, the fixing means being configured to fix the absorbent article to another article, the opening forming means being configured to form an opening, when the absorbent article is disposed of, and when viewed in the thickness direction, the opening-forming means being provided at a position not overlapping the absorbent core, and the opening-forming means and the fixing means having an overlapping portion.

According to Aspect 3, by using the fixing means provided in a portion overlapping the opening-forming means when the absorbent article is disposed of, the rolled shape of the absorbent article can be easily maintained without providing the post-processing tape for disposing of the absorbent article, thereby reducing the amount of resources used and facilitating prevention of an increase in waste.

### (Aspect 4)

The absorbent article according to any one of Aspects 1 to 3, wherein the opening-forming means is a perforation.

According to Aspect 4, an opening can be easily formed in the absorbent article, and the rolled shape of the absorbent article can be easily maintained without providing a separate member for disposing of the absorbent article, thereby reducing the amount of resources used and facilitating prevention of an increase in waste.

### (Aspect 5)

The absorbent article according to any one of Aspects 1 to 4, wherein at least one of a length of the opening-forming means in the front-back direction or a length thereof in the width direction is 1/2 or more of a maximum length of the absorbent core in the width direction.

According to Aspect 5, when at least a portion of the absorbent core is inserted into the opening to thereby maintain the rolled shape of the absorbent article at the time of disposal of the absorbent article, insertion of at least a portion of the absorbent core into the opening formed by the opening forming means is facilitated. Further, when the rolled shape of the absorbent article is maintained using the fixing means provided at a position overlapping the opening when viewed in the thickness direction at the time of disposal of the absorbent article, maintenance of the rolled shape of the absorbent article by the fixing means is facilitated.

### (Aspect 6)

The absorbent article according to any one of Aspects 1 to 5, wherein at least one of a length of the opening-forming means in the front-back direction or a length thereof in the width direction is 1/4 or more of a maximum length of the absorbent core in the front-back direction.

According to Aspect 6, when at least a portion of the absorbent core is inserted into the opening to thereby maintain the rolled shape of the absorbent article at the time of disposal of the absorbent article, insertion of at least a portion of the absorbent core into the opening formed by the opening forming means is facilitated. Further, when the rolled shape of the absorbent article is maintained using the fixing means provided at a position overlapping the opening when viewed in the thickness direction at the time of disposal of the absorbent article, maintenance of the rolled shape of the absorbent article by the fixing means is facilitated.

### (Aspect 7)

The absorbent article according to any one of Aspects 1 to 6, wherein in the front-back direction, the opening-forming means is provided on an outer side with respect to an end of the absorbent core on one side in the front-back direction.

According to Aspect 7, when the absorbent article is disposed of, the rolled shape can be easily maintained after the absorbent article has been rolled in the front-back direction.

### (Aspect 8)

The absorbent article according to Aspect 7, wherein in the front-back direction, the opening-forming means is further provided on an outer side with respect to an end of the absorbent core on an other side in the front-back direction as well.

According to Aspect 8, regardless of whether the absorbent article has been rolled up from the front side or the back side, the rolled shape of the absorbent article is easily maintained by the opening formed by the opening-forming means.

### (Aspect 9)

The absorbent article according to Aspect 7 or 8, wherein on one side in the width direction, an end of the opening-forming means located farthest on one side is provided on an outer side with respect to an end of the absorbent core located farthest on the one side in the width direction.

According to Aspect 9, when at least a portion of the absorbent core is inserted into the opening to thereby maintain the rolled shape of the absorbent article at the time of disposal of the absorbent article, insertion of at least a portion of the absorbent core into the opening formed by the opening forming means is facilitated. Further, when the rolled shape of the absorbent article is maintained using the fixing means provided at a position overlapping the opening when viewed in the thickness direction at the time of disposal of the absorbent article, maintenance of the rolled shape of the absorbent article by the fixing means is facilitated.

### (Aspect 10)

The absorbent article according to Aspect 7 or 8, wherein in the width direction, an end of the opening-forming means located farthest on one side is provided on an inner side with respect to an end of the absorbent core located farthest on the one side.

According to Aspect 10, it is possible to reduce the risk that the opening becomes excessively large due to the opening-forming means and the risk that the opening formed by the opening-forming means reaches the end of the absorbent article on the one side in the width direction.

### (Aspect 11)

The absorbent article according to any one of Aspects 7 to 10, further including: a pair of leak-proof wall portions on two sides in the width direction, wherein the leak-proof wall portions each includes a leak-proof wall elastic member that stretches and contracts in the front-back direction, a rising portion capable of rising toward a skin side, and a fixed portion not capable of rising, the fixed portion including a first fixed portion and a second fixed portion, the first fixed portion being provided on an outer side in the width direction with respect to the rising portion, the second fixed portion being provided on an outer side in the front-back direction with respect to the rising portion, in the front-back direction, the opening-forming means and the second fixed portion have an overlapping portion, and in the width direction, an end of the opening-forming means on one side in the width direction is provided on an inner side with respect to an end of the second fixed portion on the one side in the width direction, the second fixed portion being provided on the one side in the width direction.

According to Aspect 11, since the fixed portion is likely to have higher stiffness than surrounding portions, the second fixed portion can reduce the risk that the opening becomes excessively large due to the opening-forming means and the risk that the opening formed by the opening-forming means reaches the side end of the absorbent article on the one side in the width direction.

### (Aspect 12)

The absorbent article according to any one of Aspects 1 to 6, wherein the opening-forming means is provided on an outer side in the width direction with respect to a side end of the absorbent core.

According to Aspect 12, by using the opening-forming means when the absorbent article is disposed of, the rolled shape of the absorbent core can be easily maintained, thereby reducing the amount of resources used and facilitating prevention of an increase in waste.

### (Aspect 13)

The absorbent article according to Aspect 12, further including: a pair of leak-proof wall portions on two sides in the width direction, wherein the leak-proof wall portions each includes a leak-proof wall elastic member that stretches and contracts in the front-back direction, a rising portion capable of rising toward a skin side, and a fixed portion not capable of rising, the fixed portion including a first fixed portion and a second fixed portion, the first fixed portion being provided on an outer side in the width direction with respect to the rising portion, the second fixed portion being provided on an outer side in the front-back direction with respect to the rising portion, and on one side with respect to a center in the width direction, the opening-forming means is provided on the outer side in the width direction with respect to an end of the first fixed portion on the one side in the width direction.

According to Aspect 13, it is possible to reduce the risk that excrement leaks from the opening-forming means during usage.

### (Aspect 14)

The absorbent article according to Aspect 12 or 13, further including: leg gather portions on two sides in the width direction, the leg gather portions being arranged around legs of a wearer during usage, wherein the leg gather portions include leg stretching/contracting members extending along the front-back direction, and on the one side with respect to the center in the width direction, the opening-forming means is provided on an inner side in the width direction with respect to the leg stretching/contracting members.

According to Aspect 14, contraction of the leg stretching/contracting members allows an edge of the opening configured to allow insertion of at least a portion of the absorbent core to stretch and contract, thereby facilitating insertion of the absorbent core into the opening when the absorbent article is disposed of and facilitating maintenance of the rolled shape of the absorbent article.

### (Aspect 15)

The absorbent article according to any one of Aspects 12 to 14, further including: leg gather portions on two sides in the width direction, the leg gather portions being arranged around legs of a wearer during usage, wherein the leg gather portions include leg stretching/contracting members extending along the front-back direction, and a length of the opening-forming means in the front-back direction is shorter than a length of a portion capable of stretching and contracting of the leg stretching/contracting members.

According to Aspect 15, contraction of the leg stretching/contracting members allows an edge of the opening configured to allow insertion of at least a portion of the absorbent core to stretch and contract, thereby facilitating insertion of the absorbent core into the opening when the absorbent article is disposed of and facilitating maintenance of the rolled shape of the absorbent article.

### (Aspect 16)

The absorbent article according to any one of Aspects 12 to 14, further including: leg gather portions on two sides in the width direction, the leg gather portions being arranged around legs of a wearer during usage, wherein the leg gather portions include leg stretching/contracting members extending along the front-back direction, and a length of the opening-forming means in the front-back direction is longer than a length of a portion capable of stretching and contracting of the leg stretching/contracting members.

According to Aspect 16, the opening formed by the opening-forming means can be made large, thereby facilitating insertion of the absorbent core when the absorbent article is disposed of.

### (Aspect 17)

The absorbent article according any one of Aspects 12 to 16, further including: a fixing means at a non-skin-side surface of the absorbent article, the fixing means being configured to fix the absorbent article to another article, wherein on one side in the front-back direction, at least a portion of the fixing means is provided on one side in the front-back direction with respect to the opening-forming means.

According to Aspect 17, since a portion provided with the fixing means is likely to have higher stiffness, it is possible to reduce the risk that the opening becomes excessively large due to the opening-forming means and the risk that the opening formed by the opening-forming means reaches the end of the absorbent article on the one end in the front-back direction.

### (Aspect 18)

The absorbent article according to any one of Aspects 1, 2, and 4 to 17, further including: a fixing means at a non-skin-side surface of the absorbent article, the fixing means being configured to fix the absorbent article to another article, wherein when viewed in the thickness direction, the opening-forming means and the fixing means have an overlapping portion.

According to Aspect 18, when the absorbent article is disposed of, the rolled shape of the absorbent article can be more easily maintained by using the fixing means in the portion overlapping the opening-forming means after at least a portion of the absorbent core is inserted into the opening formed by the opening-forming means,.

### (Aspect 19)

The absorbent article according to any one of Aspects 1, 2, and 4 to 17, further including: a fixing means at a non-skin-side surface of the absorbent article, the fixing means being configured to fix the absorbent article to another article, wherein when viewed in the thickness direction, the opening-forming means and the fixing means do not have an overlapping portion.

According to Aspect 19, when at least a portion of the absorbent core is inserted into the opening formed by the opening-forming means at the time of disposal of the absorbent article, the risk that the fixing means adheres to a hand is reduced, thereby facilitating formation of the rolled shape of the absorbent article.

### (Aspect 20)

The absorbent article according to any one of Aspects 3 to 17, wherein the opening-forming means includes a convex portion, and the convex portion is configured to be capable of being folded back toward a skin side when forming the opening.

According to Aspect 20, since the opening formed by the opening-forming means is the convex portion, the convex portion is turned up to form the opening and folded back toward the skin side when the absorbent article is disposed of, thereby facilitating maintenance of the rolled shape of the absorbent article with the fixing means provided at a position overlapping the opening-forming means when viewed in the thickness direction.

### (Aspect 21)

The absorbent article according to any one of Aspects 3 to 17 and 20, wherein the convex portion has a convex shape that protrudes toward a center in the front-back direction.

According to Aspect 21, since the convex portion of the opening formed by the opening-forming means has a convex shape protruding toward the center in the front-back direction, the convex portion of the opening formed by being turned up when the absorbent article is disposed of can be easily fixed to the rolled portion of the absorbent article, thereby facilitating maintenance of the rolled shape of the absorbent article.

### (Aspect 22)

The absorbent article according to any one of Aspects 3 to 17, 20, and 21, wherein when viewed in the thickness direction, an area of an overlapping portion between the opening-forming means and the fixing means is 1/2 or more of an area of the convex portion capable of being folded back toward the skin side.

According to Aspect 22, the rolled shape of the absorbent article is easily maintained by the fixing means provided in the convex portion folded back toward the skin side.

### (Aspect 23)

The absorbent article according to any one of Aspects 3 to 17, 20, and 22, wherein when viewed in the thickness direction, the opening-forming means is provided at a position extending across a center of the absorbent article in the width direction.

According to Aspect 23, by fixing the absorbent article that has been rolled up at the time of disposal, at a central portion in the width direction of the absorbent article, using the fixing means provided at a position overlapping the opening-forming means in the thickness direction, the rolled shape of the absorbent article is easily maintained.

### === Embodiment(s) ===

The following describes embodiments of an absorbent article, taking an example of an absorbent pad for adults. However, it is not limited to the above; and it may also include, for example, a pull-on disposable diaper, a sanitary napkin, a panty liner, a shorts-type napkin, an absorbent article without any fastening tape, such as a light incontinence pad or the like. Further, a wearer of the absorbent article is not limited to an adult, and may also be a child or a pet.

The absorbent article according to the present invention may be used alone, or a plurality of absorbent articles may be used in combination. By using a plurality of absorbent articles in combination, it is possible to easily suppress leakage of excrement and simplify excretion handling by reducing the frequency of replacement of an absorbent article. For example, the absorbent pad of the present embodiment may be used by being fixed to clothing such as underwear or the like, or may be fixed to the inner surface (skin-side surface) of a tape-type diaper or pants-type diaper. Further, the tape-type or pants-type diaper to be worn in combination with the absorbent pad may be a disposable diaper, or may be a diaper formed of cloth or the like that can be repeatedly worn after washing or the like.

### === First Embodiment ===

### <<< Basic configuration of absorbent pad 1 (hereinafter, also referred to as "pad 1") >>>

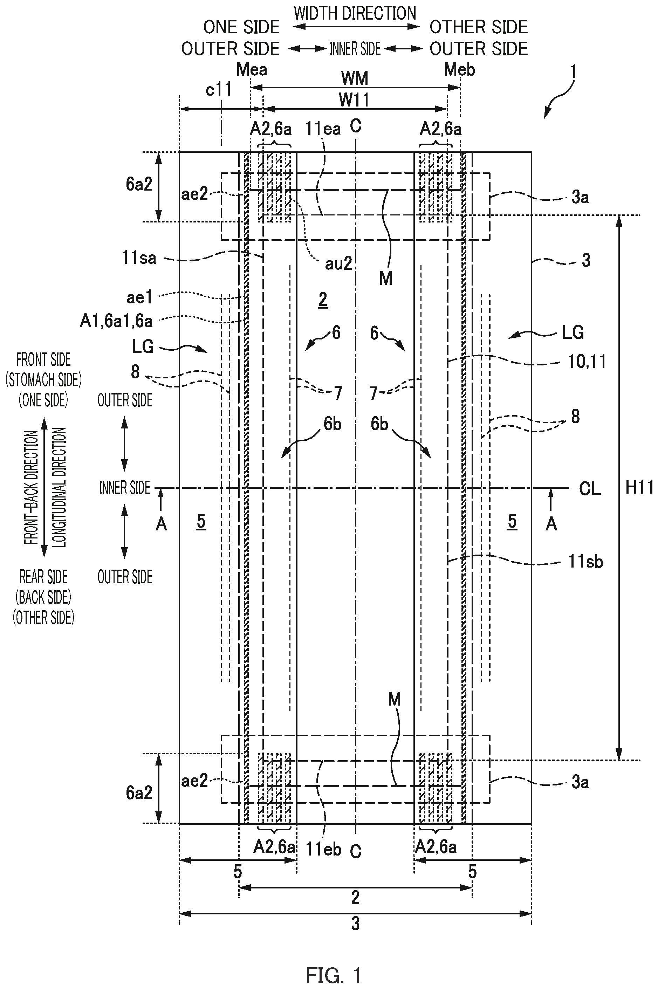

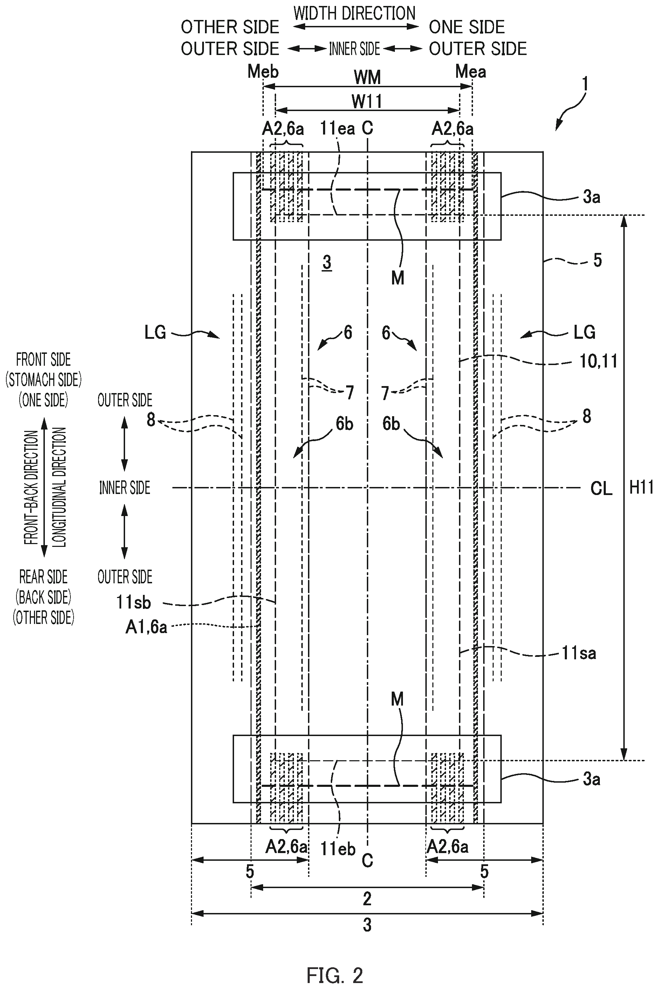

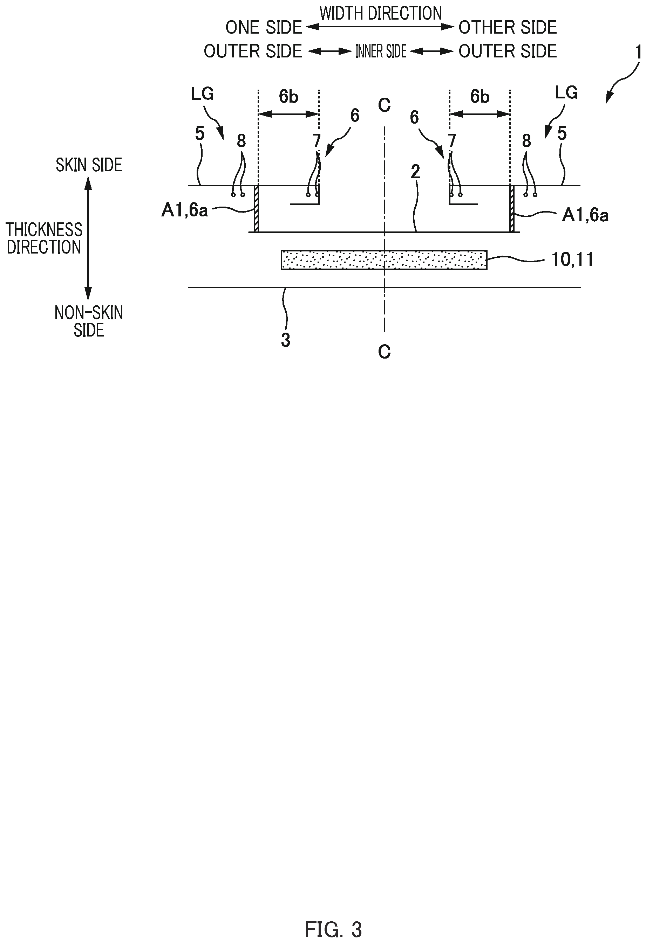

The pad 1 of the present embodiment shown in FIG. 1 and the like is in a state taken out from a container material (not shown). In general, the pad 1 is distributed and sold in a package containing one or more pads 1 in a storage material. Examples of the container material include a resin (plastic) film such as polyethylene (PE), paper, and the like. FIG. 1 is a plan view of the pad 1 when viewed from the skin side. FIG. 2 is a plan view of the pad 1 when viewed from the non-skin side. FIG. 3 is a schematic cross-sectional view taken along line A-A in FIG. 1. The pad 1, in its unfolded state, has a front-back direction, a width direction, and a thickness direction that are orthogonal to each other. In the front-back direction of the pad 1, the front side corresponds to the wearer's stomach side during usage, and the rear side corresponds to the wearer's back side. The front-back direction of the pad 1 in the unfolded state extends along the front-back direction of the absorbent body 10, and the front-back direction of the pad 1 is also referred to as "longitudinal direction." Further, in the thickness direction of the pad 1, the side that comes into contact with the wearer's skin is defined as a skin side, and the side opposite thereto is defined as a non-skin side. A center line C-C (also referred to as "center C-C") shown in FIG. 1 and the like indicates the center of the pad 1 in the width direction, and the center line CL (also referred to as "center CL") indicates the center of the pad 1 in the front-back direction. In the front-back direction of the pad 1, the center CL side in the front-back direction is defined as an inner side, and the end side of the pad 1 in the front-back direction is defined as an outer side. In the width direction of the pad 1, the center C-C side in the width direction is defined as an inner side, and the end side of the pad 1 in the width direction is defined as an outer side.

The unfolded state of the absorbent pad 1 refers to a state in which the pad 1 is entirely unfolded into a flat shape, with all fold line portions (folded parts) unfolded. When the absorbent article is pants type (shorts type), the unfolded state is defined as a state in which the side joining portions provided in the widthwise side portions of the absorbent article have been separated and laid open such that the absorbent article is unfolded into a flat shape. The stretched state of the pad 1 refers to a state in which the pad 1 has been stretched such that the wrinkles in the pad 1 can substantially no longer be seen, and a state in which the pad 1 has been stretched such that the dimensions of the constituent members of the pad 1 (for example, a side sheet 5 and the like described later) match or are close to the dimensions of such members on their own.

As shown in FIGS. 1 to 3, the pad 1 includes an absorbent body 10, a liquid-permeable top sheet 2 (for example, nonwoven fabric, perforated film, or the like) provided on the skin side with respect to the absorbent body 10, a liquid-impermeable or liquid hardly permeable back sheet 3 (for example, a resin film or the like) provided on the non-skin side with respect to the absorbent body 10, and a pair of side sheets 5 (for example, hydrophobic nonwoven fabrics or the like) respectively provided on the widthwise side portions on the skin side with respect to the top sheet 2. Note that the back sheet 3 may be constituted by a liquid-impermeable or liquid hardly permeable sheet provided on the skin side and a sheet such as a nonwoven fabric or the like provided on the non-skin side.

The absorbent body 10 includes an absorbent core 11 and a core-wrapping sheet (not shown). The absorbent core 11 may be, for example, one in which liquid-absorbent fibers such as pulp fibers containing superabsorbent polymer (SAP) are formed into a predetermined shape. Examples of the core-wrapping sheet include liquid-permeable tissue, nonwoven fabric, and the like.

The pad 1 includes a pair of leak-proof wall portions 6 in the widthwise side portions, the pair of leak-proof wall portions each including a rising portion 6b capable of rising to the skin side and a fixed portion 6a not capable of rising to the skin side. The leak-proof wall portion 6 is a so-called barrier cuff portion. The leak-proof wall portion 6 is formed of the side sheet 5 and a leak-proof wall elastic member 7, such as an elastic string or the like. The leak-proof wall elastic member 7 is fixed, in a stretched state along the front-back direction, to the folded portion of the inner end portion of the side sheet 5 in the width direction. Due to contraction of the leak-proof wall elastic member 7, the rising portion 6b of the leak-proof wall portion 6 rises to the skin side. FIG. 1 and the like illustrate a portion capable of stretching and contracting of the leak-proof wall elastic member 7. The side sheet 5 is fixed to the top sheet 2 and the back sheet 3 in an adhesion region A1 over a length from one end to the other end along its front-back direction, and two end portions in the front-back direction are fixed to the top sheet 2 in adhesion regions A2, on the inner side with respect to the adhesion regions A1 in the width direction. These adhesion regions A1 and A2 are also the fixed portions 6a that fixes the side sheet 5 to the top sheet 2 (members adjacent to the side sheet 5 in the thickness direction). Specifically, the rising portions 6b of the pad 1 are located on the inner side with respect to the adhesion regions A1 in the width direction, and on the inner side with respect to the adhesion regions A2 in the front-back direction. The fixed portions 6a include a first fixed portion 6a1 provided on the outer side with respect to the rising portion 6b in the width direction, and a second fixed portion 6a2 provided on the outer side with respect to the rising portion 6b in the front-back direction. In the leak-proof wall portion 6 of the pad 1, the first fixed portions 6a1 are portions corresponding to two ends of the adhesion region A1 in the front-back direction and the portions corresponding to the adhesion regions A2 in FIG. 1, and the second fixed portion 6a2 is a portion corresponding to the adhesion region A1.

Further, in the pad 1, a pair of leg gather portions LG that stretch and contract in the front-back direction is provided in the widthwise side portions. In the width direction, the leg gather portions LG are located on the outer side with respect to the leak-proof wall portions 6 and are arranged around the wearer's legs during usage, respectively. The leg gather portions LG are formed of the side sheets 5 and leg elastic members 8 such as elastic strings or the like. The leg elastic members 8 are fixed in a stretched state between the side sheet 5 and the back sheet 3 along the front-back direction. FIG. 1 and the like illustrate portions that can stretch and contract of the leg elastic members 8. Due to contraction of the leg elastic members 8, the leg gather portions LG fit around the wearer's legs.

The configuration of the pad 1 is not limited to the above. For example, the absorbent core 11 of the absorbent body 10 may include one layer or multiple layers. The absorbent core 11 may or may not have a slit, a recessed portion that is recessed in the thickness direction, or a protruding portion that protrudes in the thickness direction. The absorbent core 11 may include a compressed portion that is compressed in the thickness direction. The absorbent core 11 may not be covered with a core-wrapping sheet. Further, the absorbent core may be an SAP sheet in which an SAP layer is adhered to a hydrophilic sheet, an air-laid sheet in which liquid-absorbent fibers are formed into a sheet by an air-laid method, or the like. The Pad 1 may not include the leak-proof wall portions 6 or the leg gather portions LG.

The Pad 1 is provided with an adhesive portion 3a in the non-skin-side surface. The adhesive portion 3a is a means for fixing the pad 1 to another article. "Another article" can include, for example, a tape-type or pants-type disposable diaper, another absorbent article different from the pad 1 such as an absorbent pad or the like, or clothing such as wearer's underwear or the like. The adhesive portion 3a is an adhesive portion provided with an adhesive such as a hot-melt adhesive or the like, and allows the pad 1 to be repeatedly fixed to and detached from another article. The adhesive portion 3a may be applied with an adhesive such as a hot-melt adhesive without gaps over its entire region as shown in FIG. 2 and the like, or it may be applied with an adhesive in a predetermined pattern such as a so-called Ω-shaped pattern, a spiral pattern, or the like. The fixing means is not limited to the adhesive portion 3a, such as a hot-melt adhesive as in the present embodiment. For example, the fixing means may have a male member of a hook-and-loop fastener and engage with another article having a female member or the like that can engage with the male member.

### <<< Disposal handling of pad 1 >>>

Absorbent articles such as the pad 1 after wearing (after use) and the like have absorbed excrement such as urine, feces, and the like. Accordingly, in disposal handling of the absorbent article after use, it is preferable to perform the disposal so as not to touch the excrement or cause leakage of the excrement, and thus there are many cases where the absorbent article is disposed of in a rolled or folded state. It is widely known that absorbent articles includes tape (post-handling tape) in order to maintain a rolled (wound) state of the absorbent article, and a state in which the used absorbent article has been rolled or folded with the skin-side surface thereof facing inward is secured and maintained by this tape. Such a tape for maintaining the rolled state of the absorbent article does not directly contribute to the absorption of excrement, which is an original purpose of the absorbent article.

Further, in recent years, in order to achieve Sustainable Development Goals (SDGs), efficient use of resources, reduction in waste, and the like have been desired in industry. Disposing of the used absorbent article in a compact form by rolling it up or the like contributes to reduction in the amount of waste.

However, a tape provided to maintain the absorbent article in a rolled (wound) state is intended solely for disposal of the absorbent article. The amount of resources used and the amount of waste increases by the amount corresponding to such tapes. In other words, additional waste is generated for the purpose of disposing of the absorbent articles.

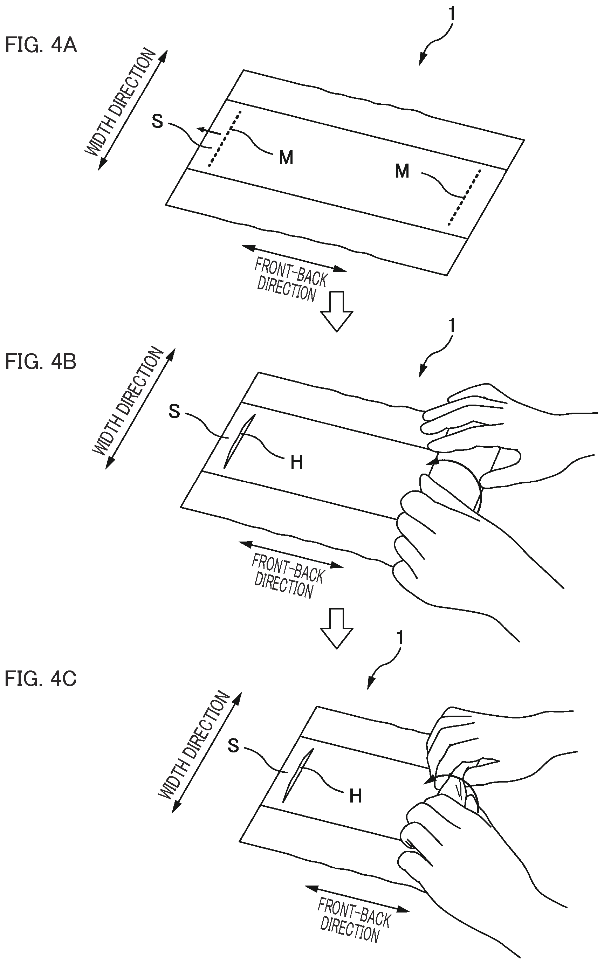

In contrast, as shown in FIGS. 1 and 2, the pad 1 of the present embodiment include neither a fastening tape nor a post-handling tape. The pad 1 further includes an opening-forming means for forming an opening H (see FIG. 4B and the like) when the pad 1 is disposed of. The opening H is an opening configured to allow insertion of at least a portion of the absorbent core 11 when the pad 1 is disposed of, and has a feature that the opening-forming means is provided at a position that does not overlap the absorbent core 11 when the pad 1 is viewed in the thickness direction.

The fastening tape is a tape member used when putting on (wearing) a so-called tape-type disposable diaper (absorbent article), and is an indispensable member for wearing the tape-type disposable diaper. In the case of a tape-type disposable diaper, generally, when the diaper is disposed of, the rolled form can be maintained by the fastening tape after the diaper is rolled. Thus, an absorbent article including a fastening tape is less likely to achieve the effects of the present invention. Further, since the fastening tape is a member having relatively high stiffness among members constituting the absorbent article, the absence of the fastening tape in the absorbent article of the present invention can reduce the risk that the stiffness of the absorbent article becomes high.

The post-handling tape is a tape member used when the absorbent article (pad 1) is disposed of, and is used for maintaining the rolled state of the absorbent article after use. Examples of the post-handling tape includes a film-shaped sheet including an adhesive portion on one surface.

Note that in the present invention, the "rolled state" of the absorbent article (pad 1) refers to a state in which the absorbent article has been rolled from one end thereof in a predetermined direction toward the other end thereof with the skin-side surface thereof facing inward or wound, so as to form a substantially circular (roll-shape) shape, as viewed from a side surface of the absorbent article, or a state in which the absorbent article folds or is folded with the skin-side surface thereof facing inward.

The "opening-forming means" refers to a means for forming an opening when the pad 1 (absorbent article) is disposed of. In the pad 1 of the present embodiment, the opening-forming means is a perforation M. The perforation M includes penetrating portions having a predetermined length and penetrating in the thickness direction from the skin-side surface to the non-skin-side surface of the pad 1, and non-penetrating portions having a predetermined length and not penetrating therethrough, the penetrating portions and the non-penetrating portions being alternately arranged in a predetermined direction. The perforation M of the present embodiment is provided on one side (front side) in the front-back direction and is a linear perforation extending along the width direction. Since the opening-forming means is the perforation M, it is possible to facilitate forming (constituting) the opening H at the time of disposal of the pad 1, while reducing the risk of impairing the function of the absorbent article in the pad 1 during usage. The easily formed opening H makes it easier to maintain the rolled shape of the pad 1, without providing a separate member (such as the post-handling tape) for disposal of the pad 1, thereby reducing the amount of resources used and facilitating prevention of an increase in waste. Further, since the opening-forming means is the perforation M, it is possible to reduce the risk that excrement leaks from the opening H during usage, and also to easily provide the opening-forming means in a manufacturing process of the pad 1. However, the opening-forming means is not limited to the perforation M. The opening-forming means may be such that the opening has already been formed in advance before use.

The "opening" is a means formed to allow insertion of at least a portion of the absorbent core 11 of the used pad 1 when the pad 1 is disposed of. Thus, the opening H has a size (area) to allow insertion of at least a portion of the absorbent core 11. Note that the opening may be an opening penetrating in the thickness direction from the skin-side surface to the non-skin-side surface of the pad 1, or may be an opening penetrating only the skin-side member (top sheet 2) of the pad 1 in the thickness direction without penetrating to the back sheet 3. The opening H of the pad 1 is a portion that has been expanded from the perforation M and is an annular space.

If the perforation M is provided at a position overlapping the absorbent core 11 when the pad 1 is viewed in the thickness direction, excrement may leak from the perforation M, or the shape of the absorbent core 11 may collapse starting from the perforation M, which may prevent obtainment of desired absorption performance. Accordingly, with the provision of the perforation M at a position not overlapping the absorbent core 11 when the pad 1 is viewed in the thickness direction, it is possible to reduce the risk that the excrement-absorption performance of the pad 1 is degraded or that excrement leaks from the pad 1, due to the perforation M.

In the pad 1 of the present embodiment, the perforation M is provided on the outer side, in the front-back direction, with respect to an end 11ea of the absorbent core 11 on one side (front side) in the front-back direction. Accordingly, when the pad 1 is rolled up along the longitudinal direction from the other side (back side) in the front-back direction at the time of disposal of the pad 1, a rolled portion of the pad 1 can be inserted into the opening H formed by the perforation M provided on the outer side in the front-back direction with respect to the end 11ea of the absorbent core 11 on the one end in the front-back direction, thereby facilitating maintenance of the rolled shape of the pad 1.

Furthermore, as in the pad 1, it is preferable that the perforation M is provided also on the outer side, in the front-back direction, with respect to an end 11eb of the absorbent core 11 on the other side (back side) in the front-back direction. That is, it is preferable that the perforations M are respectively provided on the outer sides with respect to the two ends of the absorbent core 11 in the front-back direction. By cutting the perforation M provided on the outer side in the front-back direction with respect to the end 11eb of the absorbent core 11 on the other side in the front-back direction, the opening H can also be formed on the other side (back side) of the pad 1, as in the case where it is provided on the outer side in the front-back direction with respect to the end 11ea of the absorbent core 11 on the one side (front side) in the front-back direction. Accordingly, regardless of whether the pad 1 is rolled up from the one side (front side) or the other side (back side) in the front-back direction at the time of disposal of the pad 1, the rolled shape of the pad 1 can be easily maintained with the opening H formed by the perforation M.

The following describes, with reference to FIGS. 4 to 6, a procedure for bringing the pad 1 into a disposal state when the pad 1 is disposed of. FIGS. 4 to 6 are diagrams illustrating the procedure for bringing the pad 1 into the disposal state. Note that the following procedure is merely an example of the procedure for bringing the pad 1 into the disposal state, and is not necessarily limited thereto as long as a similar method is employed. For example, the disposal state of the pad 1 may be formed by folding the pad 1 a plurality of times.

At the time of disposal of the pad 1, first, as shown in FIG. 4A, by cutting or tearing the perforation of the perforation M (opening-forming means) located on one side (for example, front side) in the front-back direction of the pad 1 in an unfolded state with the skin-side surface thereof facing upward, the pad 1 with the opening H (see FIG. 4B and the like) formed is obtained. That is, when the opening-forming means (perforation M) provided in the pad 1 is not an opening having already been formed, the opening H penetrating from the skin-side surface to the non-skin-side surface is formed starting from the perforation M. Specifically, by gripping and pulling a portion S located on the outer side in the front-back direction with respect to the perforation M provided on the one side of the pad 1 in the front-back direction, the opening H is formed with the perforation M as a starting point.

Subsequently, as shown in FIGS. 4B and 4C, the pad 1 is rolled (wound) into a rolled shape from the other side (for example, back side) of the pad 1 in the front-back direction with the skin-side surface thereof facing inward.

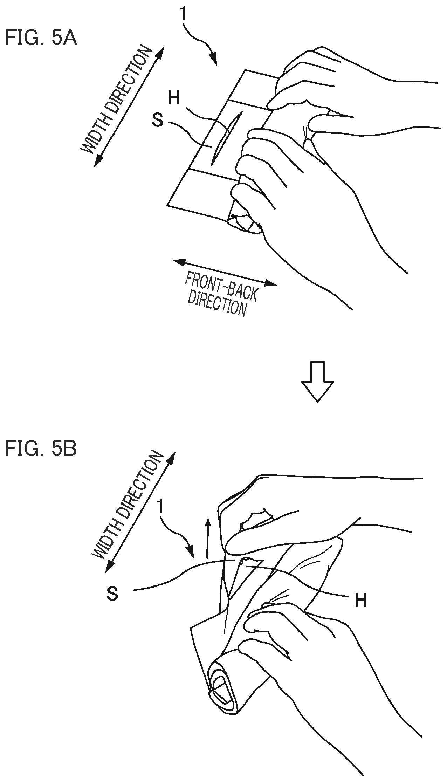

Then, as shown in FIG. 5A, when a rolled portion reaches the vicinity of the opening H, as shown in FIG. 5B, the portion S located on the outer side in the front-back direction with respect to the opening H in the pad 1 is gripped and pulled upward, thereby expanding the opening H in the front-back direction.

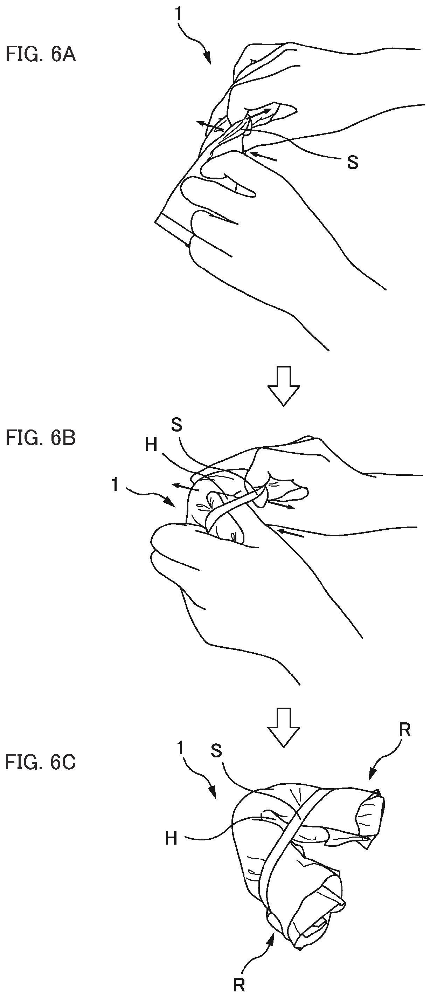

Thereafter, as shown in FIGS. 6A and 6B, while expanding the size of the opening H by pulling the portion S on the outer side in the front-back direction with respect to the opening H in the pad 1, the rolled portion of the pad 1 is inserted into the opening H. That is, a portion of the rolled portion of the pad 1 is passed through the opening H. In this event, insertion of at least a portion of the absorbent core 11 into the opening H brings a state in which at least a portion of the pad 1 has been inserted into the opening H. In the absorbent article such as the pad 1 or the like, the absorbent core 11 is thicker and has higher stiffness than other members, and thus, by inserting at least a portion of the absorbent core 11 into the opening H, it is possible to easily maintain the rolled shape of the pad 1.

Finally, by releasing the portion S located on the outer side in the front-back direction with respect to the opening H of the pad 1 from a pulled state, the pad 1 is brought into the disposal state as shown in FIG. 6C. As shown in FIG. 6C, the pad 1 is maintained in a state in which the pad 1 has been folded at substantially the center in the width direction while maintaining the rolled state. The pad 1 in the disposal state is in a state in which an annular portion of the opening H has wound around the rolled portion of the pad 1, and the rolled portion of the pad 1 inserted inside the opening H of the annular portion is restricted from being unrolled or opened. The pad 1 in this disposal state can maintain the state in which the pad 1 has been rolled up, and maintaining the pad 1 in a compact form without applying an external force. Thus, it is possible to reduce the amount of waste to be disposed of. Note that, from the state of the pad 1 shown in FIG. 6C, furthermore, portions R at two ends in the width direction of the pad 1 that are not inserted into the opening H may be inserted into the opening H (not shown). By doing so, the pad 1 can be disposed of in a more compact form, thereby being able to contribute to reduction in waste.

As such, the pad 1 can maintain the rolled form by using the opening formed by the perforation M (opening-forming means) at the time of disposal of the pad 1, even without including the post-handling tape to be used for disposing of the absorbent article. Further, since the perforation M is provided at a position that does not overlap the absorbent core 11 when the pad 1 is viewed in the thickness direction, impairment of the function of the absorbent article, such as leakage of excrement, or the like, is less likely to occur. Thus, the pad 1 can reduce the amount of resources used and prevent an increase in waste by not including the post-handling tape while ensuring the function as the absorbent article. Further, it becomes possible to reduce the manufacturing cost of the pad 1 by an amount corresponding to not using the post-handling tape. Even without using the post-handling tape, the rolled state of the pad 1 can be maintained with the opening formed by the opening-forming means, thereby being able to also contributing to reduction in waste due to achievement of a compact form.

In the procedure for bringing the pad 1 into the disposal state according to the present embodiment, the opening H is first formed when the pad 1 in which the opening has not been formed beforehand is disposed of; however, it is not limited thereto. Formation of the opening H with the perforation M (opening-forming means) as a starting point may be performed at any stage before inserting the pad 1 (absorbent core 11) into the opening H (FIG. 6A).

Furthermore, in the case where the opening-forming means is the perforation M as in the pad 1, the size of the opening H of the annular portion can be adjusted in accordance with operability in the disposal procedure and the size needed for maintaining the rolled state of the pad 1. That is, the pad 1 can adjust the size of the opening H by adjusting a range (length) to be cut in the perforation M.

Further, in the present embodiment, the opening H formed of the perforation M is assumed to completely penetrate from the skin-side surface to the non-skin-side surface of the pad 1; however, it is not limited thereto. For example, the opening H may penetrate only the top sheet 2 in the thickness direction. When having the opening H that penetrates only the top sheet 2 in the thickness direction, the pad 1 is to be rolled up to a predetermined position after forming the opening H in the same manner as in the above-described disposal procedure (FIGS. 4A to 5A). Thereafter, by inserting the rolled portion of the pad 1 into the opening H of the top sheet 2, the rolled portion of the pad 1 may be accommodated between the top sheet 2 and the back sheet 3. This makes it possible to maintain the rolled shape of the pad 1 and keep the pad 1 in a compact form, which facilitates prevention of an increase in waste, as compared with the case where the post-handling tape is used, thereby facilitating reduction in the amount of waste.

Further, the pad 1 does not include a material having 60 g/m² or more, and includes the opening-forming means for forming the opening H (see FIG. 4B and the like) when the pad 1 is disposed of. The opening H is an opening configured to allow insertion of at least a portion of the absorbent core 11 when the pad 1 is disposed of, and has a feature that the opening-forming means is provided at a position that does not overlap the absorbent core 11 when the pad 1 is viewed in the thickness direction. Even in the pad 1 having such a feature, the pad 1 can be brought into a disposal state by the same procedure as described above.

A material having 60 g/m² or more refers to, for example, a member having a relatively high basis weight (weight per unit area), such as a nonwoven fabric, a film, a fastening tape, a post-handling tape or the like. A material having a basis weight of less than 60 g/m² refers to, for example, a sheet-shaped member having a thickness of 2 mm or less, and does not include the absorbent core 11. A material having a basis weight of 60 g/m² or more has higher stiffness than a material having a basis weight of less than 60 g/m².

Accordingly, since the pad 1 does not include the material having 60 g/m² or more, flexibility of the pad 1 can be maintained as compared with the case of including the material having 60 g/m² or more. Thus, it is possible to reduce the risk that the pad 1 is unrolled to return from the rolled shape, as compared with the case where the pad 1 includes the material having 60 g/m² or more, while reducing discomfort during usage. Further, when the pad 1 is disposed of as in the above-described disposal procedure, the rolled form of the pad 1 can be maintained by using the opening formed by the perforation M (opening-forming means). Further, since the perforation M is provided at a position that does not overlap the absorbent core 11 when the pad 1 is viewed in the thickness direction, the impairment of the function of the absorbent article, such as leakage of excrement, or the like, is less likely to occur. As such, the pad 1 ensures the function as an absorbent article, and prevents an increase in waste by maintaining a compact form at the time of disposal while maintaining flexibility. Further, since the rolled state of the pad 1 can be maintained by the opening formed by the opening-forming means, it is possible to reduce the risk of leakage of excrement and soiling hands at the time of disposal, and also contribute to reduction in waste due to achievement of a compact form.

Further, since the pad 1 does not include the material having 60 g/m² or more, the amount of resources used in manufacturing the pad 1 can be reduced, and in association therewith, the amount of waste to be processed can be reduced, as compared with the case of including the material having 60 g/m² or more. Further, it is possible to contribute to reduction in energy in processing the pad 1 as waste. In particular, when a material formed of resin compounds such as a tape member (fastening tape, post-handling tape, etc.), a film, or the like used in an absorbent article is processed as waste, a high amount of energy is needed to decompose such material, and thus it tends to impose an environmental burden. Since the pad 1 does not include a material formed of resin compounds such as a tape member (fastening tape, post-handling tape, etc.), a film, or the like, having 60 g/m² or more, it becomes easier to process the pad 1 as waste with lower energy, as compared with the case of including the material having 60 g/m² or more, thereby reducing environmental burden and facilitating contribution to achievement of Sustainable Development Goals (SDGs).

Regarding the opening-forming means, it is preferable that a length WM of the perforation M (opening-forming means) in the width direction is 1/2 or more of a maximum length W11 of the absorbent core 11 in the width direction (WM ≥ W11 × 1/2). Specifically, as shown in FIG. 1, the length WM in the width direction refers to a length from an end Mea of the perforation M located farthest on one side (left side) in the width direction to an end Meb thereof located farthest on the other side (right side) in the width direction. As described in the above disposal procedure, the pad 1 is brought into a disposal state by inserting at least a portion of the pad 1 (absorbent core 11) in the rolled state into the opening H. Thus, it is preferable that the perforation M and the opening H formed of the perforation M have a predetermined size, and that the opening H is larger than the length obtained by stacking the thicknesses of the absorbent core 11 a predetermined number of times (a plurality of times, for example, 3 to 5 times). Since the length WM of the perforation M in the width direction is 1/2 or more of the maximum length W11 of the absorbent core 11 in the width direction, the opening H formed by the perforation M can be more easily expanded, as compared with the case where the length WM is less than 1/2 of the maximum length W11 of the absorbent core 11 in the width direction, thereby facilitating insertion of at least a portion of the pad 1 (absorbent core 11) in the rolled state into the opening H.

Further, as the opening-forming means, it is preferable that the length WM of the perforation M (opening-forming means) in the width direction is 1/4 or more of a maximum length H11 of the absorbent core 11 in the front-back direction (WM ≥ H11 × 1/4). Since the length WM of the perforation M in the width direction is 1/4 or more of the maximum length H11 of the absorbent core 11 in the front-back direction, the opening H formed by the perforation M can be more easily expanded, as compared with the case where the length WM of the perforation M in the width direction is less than 1/4 of the maximum length H11 of the absorbent core 11 in the front-back direction, thereby facilitating insertion of at least a portion of the pad 1 (absorbent core 11) in the rolled state into the opening H.

Further, in the case where the perforation M is provided, as in the pad 1, on the outer side (for example, front side) in the front-back direction with respect to the end 11ea of the absorbent core 11 on the one side in the front-back direction, it is more preferable that, in the width direction, the end Mea of the perforation M located farthest on the one side (for example, the left side in FIG. 1) is provided on the outer side (one side) with respect to an end 11sa of the absorbent core 11 located farthest on the one side in the width direction. Accordingly, since the opening H formed by the perforation M can be made larger on the one side in the width direction, it becomes easier to insert a portion of the pad 1 (absorbent core 11) into the opening H as in the above-described disposal procedure. Further, not only on the one side in the width direction, since the end Meb of the perforation M located farthest on the other side (for example, the right side in FIG. 1) is provided on the outer side (the other side) in the width direction with respect to an end 11sb of the absorbent core 11 located farthest on the other side in the width direction, the opening H formed by the perforation M can be made even larger, thereby facilitating the operation of the disposal procedure.

Note that regarding the perforation M of the pad 1, although the opening H can be made larger as the end Mea of the perforation located farthest on the one side is positioned further outward (on the one side) in the width direction, the opening H will no longer be formed as a closed loop, if the end Mea of the perforation located farthest on the one side reaches the side end of the pad 1 on the one side in the width direction. If the opening H is no longer in a closed annular shape, it becomes difficult to perform the operation of accommodating the rolled portion in the opening H by the above-described disposal procedure. Thus, it is preferable that the end Mea of the perforation located farthest on the one side is spaced apart from the side end of the pad 1 on the one side in the width direction by a predetermined distance.

When the perforation M is provided, as in the pad 1, on the outer side in the front-back direction with respect to the end 11ea of the absorbent core 11 on the one end in the front-back direction, the end Mea of the perforation M located farthest on the one side in the width direction (for example, the left side in FIG. 1) may be provided on the inner side (the other side, the right side in FIG. 1) with respect to the end 11sa of the absorbent core 11 on the one end in the width direction. Accordingly, it is possible to reduce the risk that the opening H becomes excessively large by the perforation M or that the opening H formed by the perforation M reaches the side end of the pad 1 on the one side in the width direction and is no longer formed as a closed loop.

Further, the pad 1 includes a pair of leak-proof wall portions 6 on two sides in the width direction. As described above, each of the leak-proof wall portions 6 includes a leak-proof-wall elastic member 7 that can stretch and contract in the front-back direction, a rising portion 6b that can rise up toward the skin side, and the adhesion regions A1 and A2 that are the fixed portions 6a provided on the outer side in the front-back direction with respect to the rising portions 6b. The fixed portions 6a each include the first fixed portion 6a1 provided on the outer side in the width direction with respect to the rising portion 6b, and the second fixed portion 6a2 provided on the outer side in the front-back direction with respect to the rising portion 6b. When such leak-proof wall portions 6 are provided, it is preferable that, in the front-back direction, the perforation M includes a portion overlapping the second fixed portion 6a2. Further, in the width direction, it is preferable that the end Mea of the perforation M on the one side in the width direction is provided on the inner side with respect to an end ae2 on the one side in the width direction of the second fixed portion 6a2 provided on the one side in the width direction. Since the fixed portion 6a including the second fixed portion 6a2 (adhesion regions A1, A2) is a portion in which sheets constituting the leak-proof wall portion 6 are fixed by a hot-melt adhesive or the like, it has higher stiffness than surrounding portions such as the rising portion 6b. Accordingly, since the perforation M is provided at a position having a portion overlapping the second fixed portion 6a2 in the front-back direction and the end Mea of the perforation M on the one end in the width direction is provided on the inner side (the other side) in the width direction with respect to the end ae2 of the second fixed portion 6a2 on the one end in the width direction, it is possible to reduce the risk that the opening H formed starting from the perforation M becomes excessively large and to reduce the risk that the opening H exceeds the end ae2 of the second fixed portion 6a2 on the one end in the width direction and reaches the side end of the pad 1 on the one side in the width direction.

Furthermore it is more preferable that in the width direction, the end Mea of the perforation M on the one side in the width direction is provided on the outer side (one side) with respect to the end au2 on the other side in the width direction of the second fixed portion 6a2 provided on the one side in the width direction. Accordingly, by facilitating formation of the opening H formed by the perforation M as large as possible, it becomes easier to maintain the rolled shape of the pad 1, while reducing the risk that the opening H becomes excessively large due to the perforation M and reducing the risk that the opening H formed by the perforation M reaches the side end of the pad 1 on the one side in the width direction.

Further, when the perforation M is provided on the outer side in the front-back direction with respect to the end 11ea of the absorbent core 11 on the one end in the front-back direction, it is preferable, as shown in FIG. 1, that, in the width direction, the end Mea of the perforation M on the one side in the width direction is provided on the inner side (the other side) with respect to the center c11 between the side end of the pad 1 on the one side in the width direction and the side end 11sa of the absorbent core 11 on the one side in the width direction. Accordingly, as compared with the case where the end Mea of the perforation M on the one side in the width direction is provided on the outer side (one side) with respect to the center c11 between the side end of the pad 1 on the one side in the width direction and the side end 11sa of the absorbent core 11 on the one side, in the width direction, it is possible to more easily reduce the risk that the opening H by the perforation M becomes excessively large and to reduce the risk that the opening H formed by the perforation M reaches the side end of the pad 1 on the one side in the width direction.

As shown in FIGS. 1 and 2, it is preferable that the pad 1 includes, in the non-skin-side surface thereof, an adhesive portion (fixing means) 3a for fixing the pad 1 to another article (an absorbent article, such as a disposable diaper or the like, different from the pad 1, or a clothing such as underwear or the like). When such an adhesive portion 3a is included, it is preferable that, when the pad 1 is viewed in the thickness direction, the perforation M and the adhesive portion 3a have a portion overlapping each other. Accordingly, at the time of disposal of the pad 1, the rolled portion of the pad 1 can be further fixed by the adhesive portion 3a provided at a portion overlapping the perforation M when viewed in the thickness direction, after inserting the pad 1 (absorbent core 11) into the opening H formed by the perforation M. Specifically, by including the adhesive portion 3a at a portion overlapping the perforation M when viewed in the thickness direction, a fixing means such as an adhesive is provided around the opening H formed by the perforation M (for example, on the non-skin-side of the portion S in FIG. 5B and the like). Thus, when the pad 1 having been rolled to a predetermined position is inserted into the opening H according to the above-described disposal procedure, the pad 1 is not only accommodated inside the opening H of the annular portion but also fixed in such a state by the adhesive portion 3a, in the state shown in FIG. 6C, thereby making it easier to maintain the rolled shape of the pad 1.

On the other hand, in the pad 1 of the present embodiment, the perforation M and the adhesive portion 3a have a portion overlapping each other when the pad is viewed in the thickness direction; however, it is not limited thereto. The pad 1 may be configured such that the perforation M and the adhesive portion 3a do not have a portion overlapping each other when the pad 1 is viewed in the thickness direction. When the perforation M and the adhesive portion 3a have a portion overlapping each other when the pad 1 is viewed in the thickness direction, there is the risk that the adhesive portion 3a of the pad 1 adheres to a hand when inserting the pad 1 that has been rolled to a predetermined position into the opening H during the disposal procedure (FIGS. 5B, 6A, and 6B). Thus, by not including the portion in which the perforation M and the adhesive portion 3a overlap each other when the pad is viewed in the thickness direction, it is possible to reduce the risk that the adhesive portion 3a adheres to the hand even when inserting, into the opening H, the pad 1 having rolled to the predetermined position when the pad 1 is disposed of, thereby facilitating disposal handling of the pad 1 and making it easier to form the rolled shape.

### <<< Modification of First Embodiment >>>

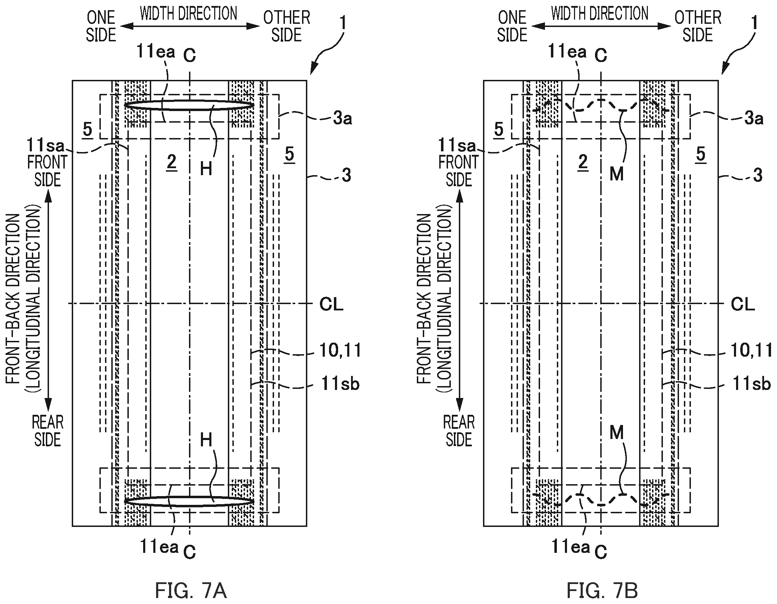

Although the pad 1 according to the first embodiment has been described above, it is not limited thereto. The opening-forming means of the pad 1 in the first embodiment is the linear perforation M extending in the width direction; however, it is not limited thereto. The shape, size, and arrangement of the opening-forming means can be set as desired. Further, the opening-forming means in the first embodiment (perforation M) is provided on each of the front side and the back side in the front-back direction; however, it is not limited thereto, and the opening-forming means (perforation M) may be provided on either one of the front side or the back side of the pad 1. Furthermore, a configuration as shown in FIG. 7 may be employed. FIG. 7 is a diagram illustrating a modification of the pad 1 of the first embodiment.

As shown in FIG. 7A, the opening-forming means of the pad 1 may be the opening H instead of the perforation M at the stage of usage (before disposal). The opening H serving as the opening-forming means shown in FIG. 7A has an elliptical shape. However, the shape of the opening H can be changed as appropriate. For example, the opening H may have any shape such as a circular shape, a rectangular shape, a polygonal shape, or the like. The opening H may be any means as long as it allows insertion of at least a portion of the pad 1 (absorbent core 11) having been rolled at the time of disposal. As shown in FIG. 7A, even when the opening-forming means of the pad 1 is the opening H, it is possible to reduce the risk that absorption of excrement is hindered and/or that excrement leaks even during usage, by providing the opening H provided on one side in the front-back direction at a position on the outer side (front side) in the front-back direction with respect to the end 11ea of the absorbent core 11 on the one side in the front-back direction.

The perforation M serving as the opening-forming means of the pad 1 is not limited to a straight line extending along the width direction. As shown in FIG. 7B, the perforation M serving as the opening-forming means of the pad 1 may have a line-shaped formation with irregularities. The perforation M shown in FIG. 7B has a wave-like shape that curves and undulates in the front-back direction. Even in the pad 1 having configurations as shown in FIGS. 7A and 7B, it is possible to maintain the rolled form of the pad 1 by using the opening H formed by the opening-forming means (perforation M, opening H) when the pad 1 is disposed of, even without including the post-handling tape used for disposal of the absorbent article. Further, since the opening-forming means is provided at a position that does not overlap the absorbent core 11 when the pad 1 is viewed in the thickness direction, there is little risk of impairing function of the absorbent article such as leakage of excrement. Thus, the pad 1 can reduce the amount of resources used and prevent an increase in waste by not including the post-handling tape while ensuring function as an absorbent article. Further, since the rolled state of the pad 1 can be maintained by the opening formed by the opening-forming means even without using the post-handling tape, it is also possible to contribute to reduction in waste by achieving a compact form.

### ===Second Embodiment===

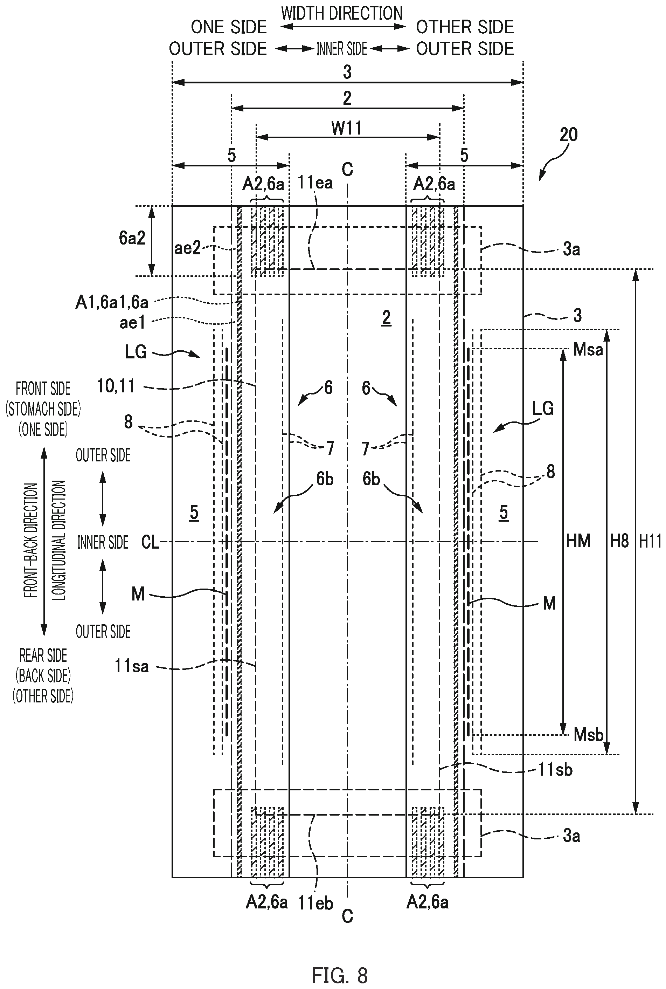

An absorbent pad 20 according to a second embodiment (hereinafter also referred to as "pad 20") will be described with reference to FIG. 8. FIG. 8 is a plan view of the pad 20 in an unfolded and stretched state as viewed from a skin side. The pad 20 has the same basic configuration as the pad 1 of the first embodiment. Thus, in the pad 20 of the present embodiment, components having the same configurations as those of the pad 1 of the first embodiment are denoted by the same reference numerals, and descriptions thereof are omitted.

### <<< Disposal handling of Pad 20 >>>

The pad 20 of the present embodiment shown in FIG. 8 and the like is in a state taken out from a container material (not shown). Generally, the pad 20 is distributed and sold in a packaged state in which one or a plurality of pads 20 are contained in a container material. Examples of the container material include a resin (plastic) film such as polyethylene (PE), paper, and the like. As shown in FIG. 8, the pad 20 of the second embodiment includes neither a fastening tape nor a post-handling tape. The pad 20 includes an opening-forming means for forming an opening at the time of disposal of the pad 20. The opening is configured to allow insertion of at least a portion of the absorbent core 11 at the time of disposal of the pad 20, and has a feature that the opening-forming means is provided at a position that does not overlap the absorbent core 11 when the pad 20 is viewed in the thickness direction.

The opening-forming means (perforation M) of the pad 20 of the present embodiment is provided on the outer side in the width direction with respect to the side ends 11sa and 11sb of the absorbent core 11. Specifically, the perforation M of the pad 20 is provided on the outer side with respect to the side end 11sa on the one side of the absorbent core 11 in the width direction, and is also provided on the outer side with respect to the side end 11sb on the other side of the absorbent core 11 in the width direction. Accordingly, when the pad 20 is rolled up from the other side (back side) in the front-back direction along the longitudinal direction when the pad 20 is disposed of, the rolled portion of the pad 20 can be inserted into the opening formed by the perforation M provided on the outer side in the width direction with respect to the side end 11sa, 11sb of the absorbent core 11 in the width direction, thereby facilitating maintenance of the rolled shape of the pad 20.

The "opening-forming means" is, as in the pad 1 of the first embodiment, a means for forming an opening when the pad 20 (absorbent article) is disposed of. The opening-forming means of the pad 20 of the present embodiment is the perforation M. The perforation M includes penetrating portions having a predetermined length and penetrating in the thickness direction from the skin-side surface to the non-skin-side surface of the pad 20, and non-penetrating portions having a predetermined length and not penetrating therethrough, the penetrating portions and the non-penetrating portions being alternately arranged in a predetermined direction. The perforation M of the present embodiment is provided on one side (left side) in the width direction and is a linear perforation extending along the front-back direction. Specifically, the perforation M is provided on each of two sides of the absorbent core 11 so as to be substantially parallel to the absorbent core 11. If an opening is provided at a position overlapping the absorbent core 11 in the front-back direction during usage, there is the risk that excrement may leak, whereas, since the opening-forming means is the perforation M, it is possible to reduce the risk of impairing function of the absorbent article in the pad 2 during usage. Further, the opening can be easily formed (configured) at the time of disposal of the pad 20 with the perforation M as a starting point.

By using the opening easily formed with the perforation M as a starting point, it becomes easier to maintain the rolled shape of the pad 20 without providing a separate member (such as the post-handling tape) for disposing of the pad 20, thereby reducing the amount of resources used and facilitating prevention of an increase in waste. Further, since the opening-forming means is the perforation M, the opening-forming means can be easily provided even in a manufacturing process of the pad 20.

The "opening" is a means formed to allow insertion of at least a portion of the absorbent core 11 of the used pad 20 when the pad 20 is disposed of. Thus, the opening has a size (area) to allow insertion of at least a portion of the absorbent core 11. Note that the opening may be an opening penetrating in the thickness direction from the skin-side surface to the non-skin-side surface of the pad 20, or may be an opening penetrating only the skin-side member (top sheet 2) of the pad 20 in the thickness direction without penetrating through the back sheet 3. The opening of the pad 20 is a portion expanded from the perforation M and is a loop-shaped space.

By providing the perforation M at a position that does not overlap the absorbent core 11 when the pad 20 is viewed in the thickness direction, it is possible to reduce the risk that an excrement absorption function of the pad 20 is degraded or that excrement leaks from the pad 20 due to the perforation M.

Although a procedure for bringing the pad 20 into a disposal state when the pad 20 is disposed of differs in part from that of the pad 1 of the first embodiment, it is substantially similar thereto. The procedure for bringing the pad 20 into the disposal state is not necessarily limited thereto as long as a similar method is employed, and for example, the disposal state of the pad 20 may be formed by folding the pad 20 a plurality of times.

When the pad 20 is disposed of, first, the perforation of the perforation M (opening-forming means) on one side in the width direction (for example, the left side in FIG. 8) of the pad 20 in an unfolded state with the skin-side surface facing upward is cut or torn, thereby obtaining the pad 20 in a state in which an opening is formed (not shown).

Subsequently, the pad 20 is rolled up in the front-back direction into a rolled shape with the skin-side surface facing inward (see FIGS. 4B to 5A).

Then, the rolled portion of the pad 20 is inserted into openings provided on two sides in the width direction of the pad 20, and when a force applied to the pad 20 to perform the disposal procedure is released, the pad 20 is maintained in the rolled state, resulting in the disposal state. The pad 20 in the disposal state is in a state in which annular portion corresponding to the opening has been wound around the rolled portion of the pad 20, and the pad 20 in the rolled state inserted inside the opening of the annular portion is restricted from being unrolled or opened. The pad 20 in this disposal state can maintain the state in which the pad 20 has been rolled and maintain the pad 20 in a compact form even without applying an external force. Thus, it is possible to reduce the amount of waste to be discharged.

As described above, the pad 20 can maintain the rolled form by using the opening that is formed by the perforation M (opening-forming means) when the pad 20 is disposed of, even without including the post-handling tape used for disposing of the absorbent article. Further, since the perforation M is provided at a position that does not overlap the absorbent core 11 when the pad 20 is viewed in the thickness direction, there is little risk of impairing function of the absorbent article such as leakage of excrement. Thus, the pad 20 can reduce the amount of resources used and prevent an increase in waste by not including the post-handling tape while ensuring function as an absorbent article. In addition, manufacturing cost of the pad 20 can be reduced by an amount corresponding to not using the post-handling tape. Further, even without using the post-handling tape, the rolled state of the pad 20 can be maintained by the opening formed by the opening-forming means, thereby also contributing to reduction in waste by achieving a compact form.

Note that in the procedure for bringing the pad 20 into the disposal state according to the present embodiment, the opening is first formed when the pad 20 not having an opening formed in advance is disposed of; however, it is not limited thereto. Formation of the opening with the perforation M (opening-forming means) as a starting point may be performed at any stage before inserting the pad 20 (absorbent core 11) into the opening H.

Furthermore, when the opening-forming means of the pad 20 is the perforation M, the size of the opening of the annular portion can be adjusted in accordance with the operability in the disposal procedure and the size needed to maintain the rolled state of the pad 20. That is, the pad 20 can adjust the size of the opening by adjusting a range (length) of the perforation M in which cutting is performed.

Further, in the present embodiment, the opening formed by the perforation M is assumed to completely penetrate from the skin-side surface to the non-skin-side surface of the pad 20; however, it is not limited thereto. For example, the opening may penetrate only the top sheet 2 in the thickness direction. When the opening penetrates only the top sheet 2, the pad 20 is rolled up to a predetermined position, after forming the opening H in the same manner as in the above-described disposal procedure. Thereafter, the rolled portion of the pad 20 may be accommodated between the top sheet 2 and the back sheet 3, by inserting the rolled portion of the pad 20 into the opening of the top sheet 2.

In the pad 20 of the present embodiment, the perforation M is provided on the outer sides with respect to the two side ends 11sa and 11sb of the absorbent core 11 in the width direction; however, it is not limited thereto. In the pad 20, the perforation M may be provided only on one of the outer side in the width direction with respect to the side end 11sa of the absorbent core 11 on the one side in the width direction or the outer side in the width direction with respect to the side end 11sb of the absorbent core 11 on the other side in the width direction. Even when the perforation M (opening forming means) is provided only on one of the outer side in the width direction with respect to the side end 11sa of the absorbent core 11 on the one side or the outer side in the width direction with respect to the side end 11sb of the absorbent core 11 on the other side, the rolled state of the pad 20 can be maintained, by inserting the rolled pad 20 into the opening H provided on either one side in the width direction.

Further, the pad 20 does not include a material having 60 g/m² or more, and includes an opening-forming means for forming an opening at the time of disposal of the pad 20, and this opening is an opening configured to allow insertion of at least a portion of the absorbent core 11 at the time of disposal of the pad 20. The pad 20 has the feature that this opening-forming means is provided at a position that does not overlap the absorbent core 11 when the pad 20 is viewed in the thickness direction. Even in the pad 20 having such a feature, the pad 20 can be brought into a disposal state by the same procedure as described above.

Accordingly, since the pad 20 does not include a material having 60 g/m² or more, flexibility of the pad 20 can be maintained, as compared with the case where a material having 60 g/m² or more is included. Thus, while reducing discomfort during usage, it is possible to reduce the risk that the pad 20 is unrolled to return from the rolled shape, as compared with the case where the pad 20 includes a material having 60 g/m² or more. Further, when the pad 20 is disposed of as in the above-described disposal procedure, the rolled form of the pad 20 can be maintained by using the opening formed by the perforation M (opening-forming means). Further, since the perforation M is provided at a position that does not overlap the absorbent core 11 when the pad 20 is viewed in the thickness direction, there is little risk of impairing function of the absorbent article such as leakage of excrement. As such, the pad 20 prevents an increase in waste by achieving a compact form at the time of disposal while ensuring function as an absorbent article and having flexibility. Further, since the rolled state of the pad 20 can be maintained by the opening formed by the opening-forming means, it is possible to contribute to reduction in waste by achieving a compact form.

Further, since the pad 20 does not include a material having 60 g/m² or more, the amount of resources used in manufacturing the pad 20 can be reduced, and in association therewith, the amount of waste to be processed can be reduced more than in the case where it includes a material having 60 g/m² or more. In addition, it is possible to contribute to reduction in the amount of energy in processing the pad 20 as waste. In particular, when materials formed of resin compounds such as a film and a tape member (a fastening tape, a post-handling tape, etc.) used for absorbent articles are processed as waste, a high amount of energy is needed to decompose such materials, and thus they are likely to impose an environmental burden. The pad 20 not including materials formed of resin compounds such as a film and a tape member (a fastening tape, a post-handling tape, etc.) having 60 g/m² or more makes it easier to process the pad 20 as waste with lower energy, as compared with the case where materials having 60 g/m² or more are included, thereby reducing environmental burden and facilitating contribution to achievement of Sustainable Development Goals (SDGs).

Regarding the opening-forming means, it is preferable that a length HM of the perforation M (opening-forming means) in the front-back direction is 1/2 or more of the maximum length W11 of the absorbent core 11 in the width direction (HM ≥ W11 × 1/2). Specifically, as shown in FIG. 8, the length HM in the front-back direction refers to a length from an end Msa of the perforation M located farthest on one side (front side) in the front-back direction to an end Msb of the perforation M located farthest on the other side (back side) in the front-back direction. As described in the above disposal procedure, the pad 20 is brought into a disposal state by inserting at least a portion of the pad 20 (absorbent core 11) in the rolled state into the opening. Thus, it is preferable that the perforation M and the opening formed thereby have a predetermined size, and that the opening is larger than a length obtained by stacking the thickness of the absorbent core 11 a predetermined times (a plurality of times, for example, 3 to 5 times). The length HM of the perforation M in the front-back direction being 1/2 or more of the maximum length W11 of the absorbent core 11 in the width direction makes it possible to expand the opening formed by the perforation M more easily, as compared with the case where the length HM of the perforation M in the front-back direction is less than 1/2 of the maximum length W11 of the absorbent core 11 in the width direction, thereby facilitating insertion of at least a portion of the rolled pad 20 (absorbent core 11) into the opening.

Further, as the opening-forming means, it is preferable that the length HM of the perforation M (opening-forming means) in the width direction is 1/4 or more of the maximum length H11 of the absorbent core 11 in the front-back direction (HM ≥ H11 × 1/4). The length HM of the perforation M in the width direction being 1/4 or more of the maximum length H11 of the absorbent core 11 in the front-back direction makes it possible to expand the opening formed by the perforation M more easily, as compared with the case where the length HM of the perforation M in the width direction is less than 1/4 of the maximum length H11 of the absorbent core 11 in the front-back direction, thereby facilitating insertion of at least a portion of the rolled pad 20 (absorbent core 11) into the opening.

Further, the pad 20 includes the leak-proof wall portions 6 in a pair on two sides in the width direction. Each of the leak-proof wall portions 6 includes the leak-proof-wall elastic member 7 that stretches and contracts in the front-back direction, the rising portion 6b capable of rising up toward the skin side, and adhesion regions A1 and A2 that are the fixed portions 6a provided on the outer side with respect to the rising portion 6b in the front-back direction. The fixed portions 6a each include the first fixed portion 6a1 provided on the outer side in the width direction with respect to the rising portion 6b, and the second fixed portion 6a2 provided on the outer side in the front-back direction with respect to the rising portion 6b. When such leak-proof wall portions 6 are provided, it is preferable that, on one side (for example, the left side) with respect to the center in the width direction, the perforation M is provided on the outer side in the width direction with respect to an end ae1 of the first fixed portion 6a1 on one side in the width direction. The leak-proof wall portions 6 are provided to block excrement so as to prevent excrement from leaking outward in the width direction in the pad 20. Accordingly, by providing the perforation M on the outer side in the width direction with respect to the end ae1 of the first fixed portion 6a1 on one end in the width direction in the leak-proof wall portion 6, it is possible to reduce the risk that excrement leaks from the perforation M to the outside of the pad 20.

As shown in FIG. 8, the pad 20 includes, on two sides in the width direction, the leg gather portions LG that are arranged around the legs of the wearer during usage. Each of the leg gather portions LG includes a leg elastic member 8 extending along the front-back direction. Further, it is preferable that, on one side with respect to the center in the width direction, the perforation M is provided on the inner side in the width direction with respect to the leg elastic member 8. When the perforation M is provided on the outer side in the width direction with respect to the side end 11sa, 11sb of the absorbent core 11, as in the pad 20, the edge of the opening formed by the perforation M allowing insertion of at least a portion of the pad 20 (absorbent core 11) can stretch and contract by contraction of the leg elastic member 8 in the front-back direction. Such stretch and contraction of the edge of the opening makes it easier to insert the absorbent core 11 into the opening, when the pad 20 is disposed of. Further, it becomes easier to maintain the rolled shape of the pad 20.

Further, when the pad 20 includes, on two sides in the width direction, the leg gather portions LG having the leg elastic members 8, it is preferable that the length HM of the perforation M in the front-back direction is shorter than a length H8 of a portion capable of stretching and contracting of the leg elastic member 8 (HM < H8). In FIG. 8, each of the leg elastic members 8 is shown as a portion capable of stretching and contracting, and the length H8 of the leg elastic member 8 is a length of the portion capable of stretching and contracting. This makes it possible to stretch and contract the edge of the opening formed by the perforation M allowing insertion of at least a portion of the pad 20 (absorbent core 11) by contraction of the leg elastic member 8 in the front-back direction. By such stretch and contraction of the edge of this opening, it becomes easier to insert the absorbent core 11 into the opening when the pad 20 is disposed of. Further, it becomes easier to maintain the rolled shape of the pad 20.

Meanwhile, when the pad 20 includes the leg gather portions LG having the leg elastic members 8 on two sides in the width direction, the length HM of the perforation M in the front-back direction may be longer than the length H8 of the portion capable of stretching and contracting of the leg elastic member 8 (HM > H8). Accordingly, the opening formed by the perforation M can be formed larger, as compared with the case where the length HM of the perforation M in the front-back direction is shorter than the length H8 of the portion capable of stretching and contracting of the leg elastic member 8, thereby facilitating insertion of at least a portion of the rolled pad 20 (absorbent core 11) into the opening when the pad 20 is disposed of.

Further, as shown in FIG. 8, when the pad 20 includes, in the non-skin-side surface thereof, an adhesive portion (fixing means) 3a for fixing the pad 20 to another article, it is preferable that, on one side in the front-back direction (for example, the front side), at least a portion of the adhesive portion 3a is provided on the one side in the front-back direction with respect to the perforation M. A portion of the pad 20 in which the adhesive portion 3a is provided is likely to have higher stiffness than a portion in which the adhesive portion 3a is not provided. Thus, when the pad 20 includes the perforation M extending in the front-back direction, it is possible to reduce the risk that the opening formed by the perforation M becomes excessively large and the risk that the opening formed by the perforation M reaches an end of the pad 20 on one side in the front-back direction, by providing at least a portion of the adhesive portion 3a having relatively high stiffness on the outer side in the front-back direction with respect to the perforation M.

Further, when the pad 20 includes, in the non-skin-side surface thereof, the adhesive portion (fixing means) 3a for fixing the pad 20 to another article (an absorbent article, such as a disposable diaper or the like, different from the pad 20, or a clothing such as underwear or the like), it is preferable that the perforation M and the adhesive portion 3a do not have a portion overlapping each other, when the pad 20 is viewed in the thickness direction. When the perforation M and the adhesive portion 3a have a portion overlapping each other when the pad 20 is viewed in the thickness direction, there is the risk that the adhesive portion 3a of the pad 20 adheres to a hand when inserting the pad 20 having been rolled to a predetermined position into the opening in the disposal procedure. Thus, the perforation M and the adhesive portion 3a not including a portion overlapping each other when the pad 20 is viewed in the thickness direction makes it possible to reduce the risk that the adhesive portion 3a adheres to the hand, even when inserting, into the opening, the pad 20 having been rolled to the predetermined position when the pad is disposed of, thereby facilitating disposal handling of the pad 20 and making it easier to form the rolled shape.

Meanwhile, it is assumed that the pad 20 of the present embodiment does not have a portion in which the perforation M and the adhesive portion 3a overlap each other when the pad 20 is viewed in the thickness direction; however, it is not limited thereto. Such a portion in which the perforation M and the adhesive portion 3a overlap each other when the pad 20 is viewed in the thickness direction may be included. Accordingly, when the pad 20 is disposed of, the rolled portion of the pad 20 can be fixed by the adhesive portion 3a provided at the portion overlapping the perforation M when viewed in the thickness direction, after inserting the pad 20 (absorbent core 11) into the opening H formed by the perforation M. Thus, when the pad 20 having been rolled according to the above-described disposal procedure is inserted into the opening, the rolled portion is not only accommodated inside the opening of the annular portion but also fixed by the adhesive portion 3a, in the disposal state, thereby making it easier to maintain the rolled shape of the pad 20.

### <<< Modification of Second Embodiment >>>

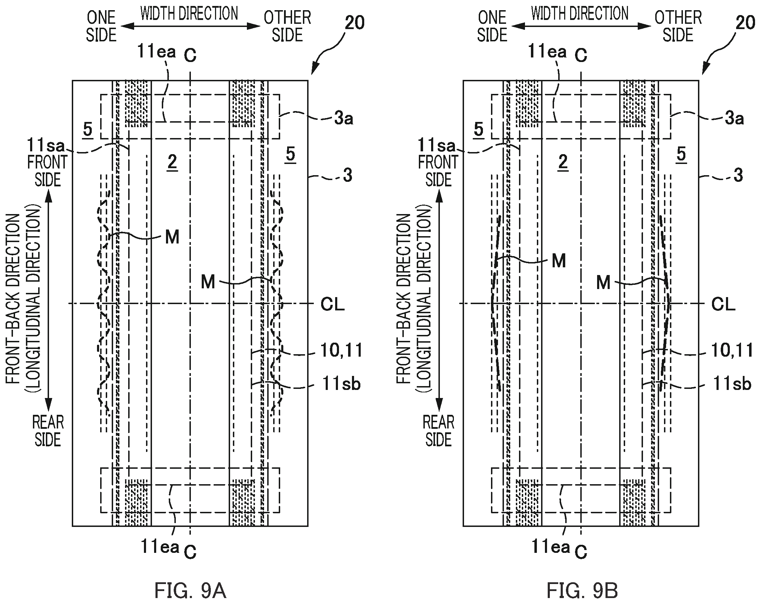

Although the pad 20 according to the second embodiment has been described above, it is not limited thereto. The opening-forming means of the pad 20 of the first embodiment is the perforation M that is linear and extends in the front-back direction; however, it is not limited thereto. The shape, size, and arrangement of the opening-forming means can be set freely. For example, a configuration as shown in FIG. 9 may be employed. FIG. 9 is a diagram illustrating a modification of the pad 20 of the second embodiment.

Modifications shown in FIGS. 9A and 9B include the perforations M serving as the opening-forming means extending in the front-back direction. The perforation M of the pad 20 shown in FIG. 9A has a wave-like shape that undulates and curves in the width direction. The perforation of the pad 20 shown in FIG. 9B has a shape in which, on one side in the width direction, a portion of the perforation at a central portion in the front-back direction protrudes toward the one side in the width direction, and portions thereof on both sides in the front-back direction are inclined toward an inner side in the width direction.

Even in the pad 20 having configurations as shown in FIGS. 9A and 9B, the rolled form of the pad 20 can be maintained by using the opening formed by the opening-forming means (perforation M) when the pad 20 is disposed of, even without including a post-handling tape for disposing of an absorbent article. Further, since the opening-forming means is provided at a position that does not overlap the absorbent core 11 when the pad 20 is viewed in the thickness direction, there is little risk of impairing function of an absorbent article such as leakage of excrement and the like. Thus, the pad 20 can reduce the amount of resources used and prevent an increase in waste by not including the post-handling tape while ensuring function as an absorbent article. Further, even without using the post-handling tape, it is possible to maintain the rolled state of the pad 20 by the opening formed of the opening forming means, thereby being able to contribute to waste reduction by achieving a compact form.

### === Third Embodiment ===

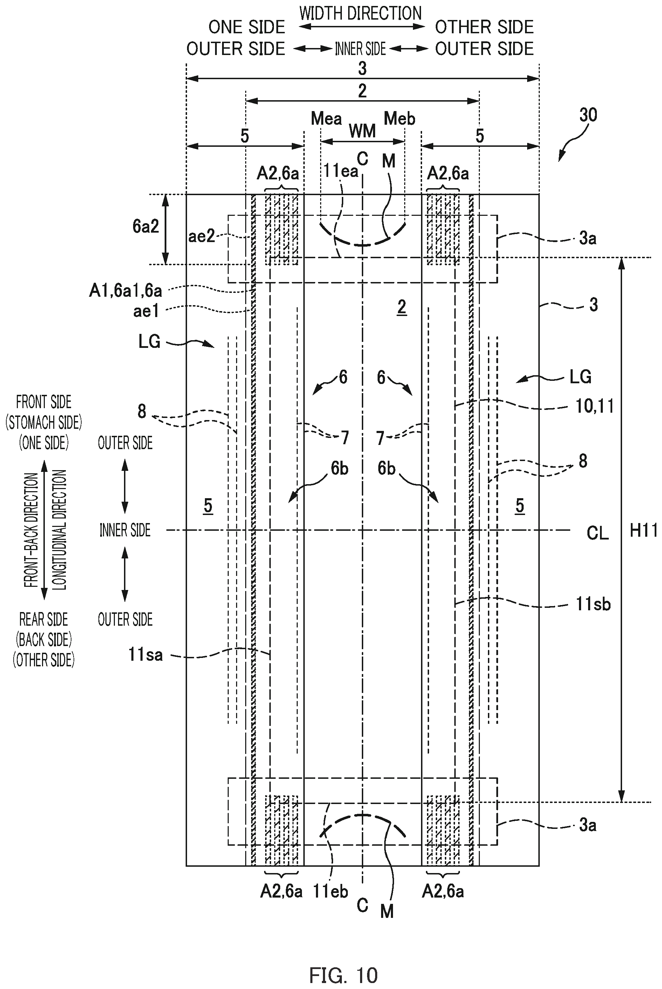

An absorbent pad 30 according to a third embodiment (hereinafter also referred to as "pad 30") will be described with reference to FIG. 10. FIG. 10 is a plan view of the pad 30 in an unfolded and stretched state as viewed from the skin side. The pad 30 has the same basic configuration as the pad 1 of the first embodiment. Thus, components of the pad 30 in the present embodiment having the same configuration as those of the pad 1 in the first embodiment are denoted by the same reference numerals, and description thereof is omitted.

### <<< Disposal handling of Pad 30 >>>

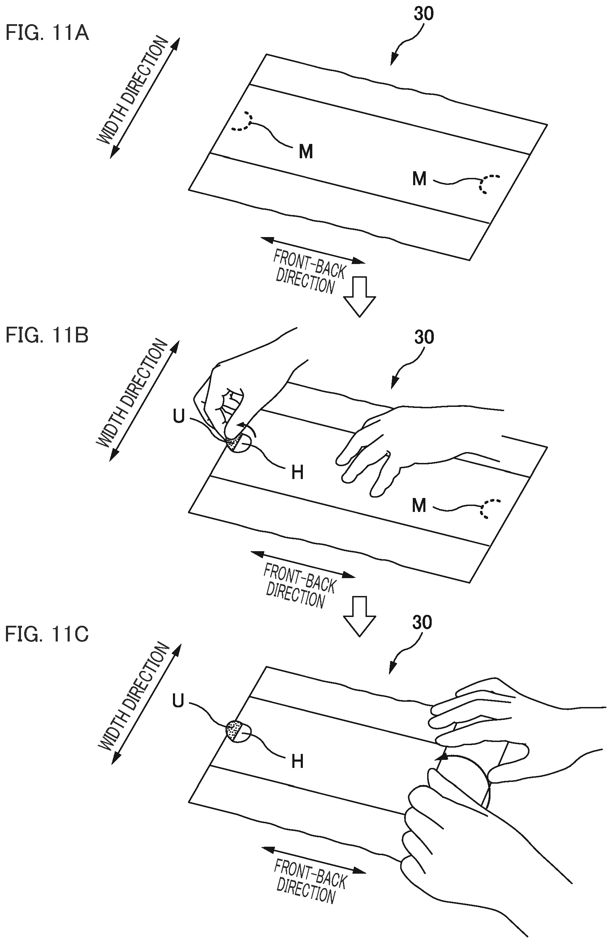

The pad 30 of the present embodiment shown in FIG. 10 and the like is in a state taken out from a container material (not shown). Generally, the pad 30 is distributed and sold in a packaged state in which one or a plurality of pads 30 are contained in a container material. Examples of the container material include a resin (plastic) film such as polyethylene (PE), paper, and the like. The pad 30 of the present embodiment does not include the post-handling tape. As shown in FIG. 10, the pad 30 includes an opening-forming means for forming an opening H (see FIG. 11B and the like) when the pad 30 is disposed of, and has the feature that this opening-forming means is provided at a position that does not overlap the absorbent core 11 when the pad 30 is viewed in the thickness direction.

The perforation M of the present embodiment is provided on one side (front side) in the front-back direction, and has a substantially semicircular shape with a convex portion directed toward a central portion in the front-back direction.

The "opening-forming means" is a means for forming an opening when the pad 30 (absorbent article) is disposed of. The opening-forming means in the pad 30 is also a region that can be folded back toward the skin side when an opening is formed at the time of disposal. The opening-forming means the pad 30 in the present embodiment is a perforation M. The perforation M includes penetrating portions having a predetermined length and penetrating in the thickness direction from the skin-side surface to the non-skin-side surface of the pad 30, and non-penetrating portions having a predetermined length and not penetrating therethrough, the penetrating portions and the non-penetrating portions being alternately arranged in a predetermined direction.

Since the opening-forming means is the perforation M, it is possible to facilitate formation of the opening H at the time of disposal of the pad 30, while reducing the risk of impairing function of an absorbent article during usage. By the easily formed opening H, it becomes easier to maintain the rolled shape of the pad 30 without providing a separate member (post-handling tape or the like) for disposing of the pad 30, thereby reducing the amount of resources used and facilitating prevention of an increase in waste. Further, the opening-forming means can be easily provided in the manufacturing process of the pad 30. However, the opening-forming means is not limited to the perforation M. The opening-forming means may be an opening that is formed in advance before usage.

The "opening" is formed so as to allow a portion corresponding thereto to be folded back such that the non-skin-side surface thereof is exposed to the skin side by forming the opening when the pad 30 is disposed of, and is a portion penetrating from the skin side toward the non-skin side. The opening H of the pad 30 is a portion expanded from the perforation M and defines a closed-loop space.

Further, if the perforation M is provided at a position overlapping the absorbent core 11 when the pad 30 is viewed in the thickness direction, there is the risk that excrement may leak from the perforation M or that the shape of the absorbent core 11 may collapse starting from the perforation M, resulting in failure to obtain a desired absorption function. Thus, by providing the perforation M at a position that does not overlap the absorbent core 11 when the pad 30 is viewed in the thickness direction, it is possible to reduce the risk that the absorption function of the pad 30 is degraded or that excrement leaks from the pad 30 due to the perforation M.

In the pad 30 of the present embodiment, the perforation M is provided on the outer side, in the front-back direction, with respect to the end 11ea of the absorbent core 11 on one side (front side) in the front-back direction. Accordingly, when the pad 30 is rolled up from the other side (back side) in the front-back direction along the longitudinal direction at the time of disposal of the pad 30, the adhesive portion 3a in the non-skin-side surface corresponding to the opening H formed by the perforation M provided on the outer side in the front-back direction with respect to the end 11ea of the absorbent core 11 on the one end in the front-back direction adheres to the non-skin-side surface of the rolled pad 30, thereby facilitating maintenance of the rolled shape of the pad 30.

Further, it is preferable that, as in the pad 30, the perforation M is also provided on the outer side, in the front-back direction, with respect to the end 11eb of the absorbent core 11 on the other side (back side) in the front-back direction. That is, it is preferable that the perforations M are respectively provided on the outer sides with respect to two ends of the absorbent core 11 in the front-back direction. By cutting the perforation M provided on the outer side in the front-back direction with respect to the end 11eb of the absorbent core 11 on the other side in the front-back direction, the opening H can be formed also on the other side (back side) of the pad 30, as in the case where the perforation M is provided on the outer side with respect to the end 11ea of the absorbent core 11 on the one side (front side) in the front-back direction. Accordingly, regardless of whether the pad 30 is rolled up from the one side (front side) or the other side (back side) in the front-back direction when the pad 30 is disposed of, the rolled shape of the pad 30 can be easily maintained by the opening H formed by the perforation M.

Further, as shown in FIG. 10, it is preferable that the perforation M (opening-forming means) has a convex portion. It is also preferable that the convex portion is configured to be foldable back toward the skin side when forming the opening H (see FIG. 11B). When disposing of the pad 30 according to a procedure described later, the convex portion is turned up to form the opening H since the opening H formed by the perforation M has the convex portion, and is folded back toward the skin side, thereby exposing the adhesive portion 3a on the skin side, and the adhesive portion 3a that is provided at a position overlapping the perforation M in the thickness direction adhering to the non-skin-side surface of the rolled pad 30 makes it easier to maintain the rolled shape of the pad 30.

Furthermore, it is preferable that the convex portion of the perforation M has a convex shape protruding toward the center CL in the front-back direction. As such, with the convex portion having a convex shape directed to the center CL, it is facilitated to fix, to the rolled portion of the pad 30, the convex portion by the opening H formed by turning it up when the pad 30 is disposed of, as in a disposal procedure described later, thereby making it easier to maintain the rolled shape of the pad 30.

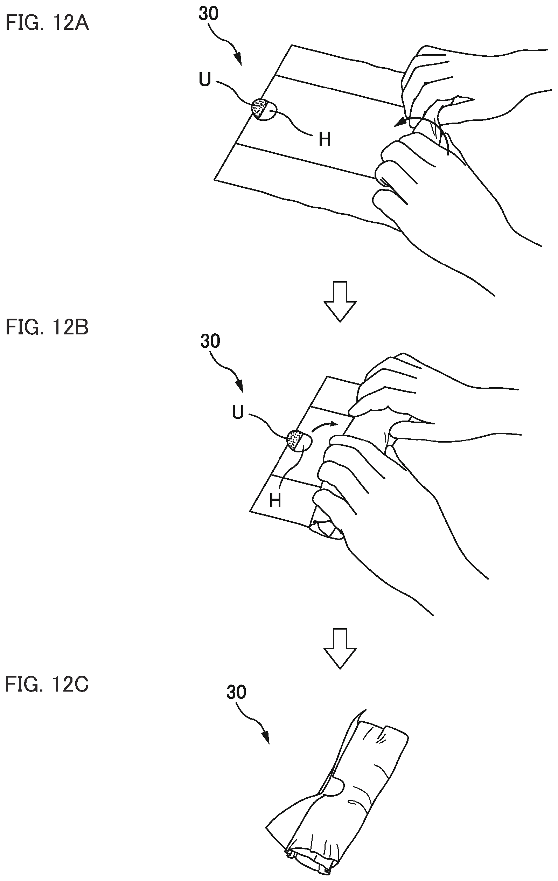

The following describes the procedure of bringing the pad 30 into the disposal state at the time of disposal of the pad 30 with reference to FIGS. 11 and 12. FIGS. 11 and 12 are diagrams illustrating the procedure of bringing the pad 30 into the disposal state. The following procedure is merely an example of the procedure for bringing the pad 30 into the disposal state, and it is not limited thereto as long as a similar method is employed. For example, the disposal state of the pad 30 may be formed by folding the pad 30 a plurality of times.

When the pad 30 is disposed of, first, in an unfolded state with the skin-side surface facing upward as shown in FIG. 11A, the perforation of the perforation M (opening-forming means) on one side in the front-back direction (for example, the front side) is cut or torn to form the opening H, and a portion cut to form the opening H is folded back toward the skin side to bring a state shown in FIG. 11B. That is, when the opening-forming means (perforation M) included in the pad 30 is not an opening formed in advance, the opening H penetrating from the skin-side surface to the non-skin-side surface of the pad 30 is formed starting from the perforation M. Note that, since the pad 30 includes the adhesive portion 3a overlapping the perforation M when the pad 30 is viewed in the thickness direction, a portion U in the non-skin-side surface of the portion folded back toward the skin side for forming the opening H is provided with a fixing means such as a hot-melt adhesive for fixing the pad 30 to another article.

Subsequently, as shown in FIG. 11C and FIG. 12A, the pad 30 is rolled into a roll shape from the other side in the front-back direction (for example, the back side) of the pad 30 with the skin-side surface facing inward.

Then, as shown in FIG. 12B, when the rolled portion reaches near the opening H, as shown in FIG. 12B, the portion U on the non-skin-side surface that has been folded back toward the skin side to form the opening H in the pad 30 is fixed, for example, by adhering to the non-skin-side surface of the rolled portion of the pad 30.

Accordingly, the pad 30 in this disposal state can maintain the state in which the pad 30 has been rolled and maintain the pad 30 in a compact form without applying an external force. Thus, the amount of waste to be discharged can be reduced.

As such, even without including the post-handling tape used for disposing of an absorbent article, the pad 30 can maintain the form in which the pad 30 has been rolled, by including the adhesive portion 3a overlapping the perforation M when viewed in the thickness direction and using the adhesive portion 3a in the non-skin-side surface corresponding to the opening H folded back toward the skin side, when the pad 30 is disposed of. The adhesive portion 3a is a function needed to fix the pad 30 to underwear or another absorbent article, that is, a function needed to use the pad 30 as an absorbent article. By using this adhesive portion 3a, which is provided to exhibit such function as an absorbent article, at the time of disposal, the pad 30 can be brought into the disposal state without newly providing a member for disposal, such as a post-handling tape. Thus, the pad 30 can reduce the amount of resources used and prevent an increase in waste by not including the post-handling tape while ensuring the function as an absorbent article. Further, the manufacturing cost of the pad 30 can be reduced accordingly by an amount corresponding to not using the post-handling tape. In addition, since the perforation M is provided at a position that does not overlap the absorbent core 11 when the pad 30 is viewed in the thickness direction, there is little risk of impairment the function as an absorbent article, such as leakage of excrement and the like. Thus, even without using the post-handling tape, it is possible to maintain the rolled state of the pad 30 by the opening formed by the opening-forming means, thereby being able to contribute to reduction in waste by achieving a compact form.

Note that in the procedure of bringing the pad 30 into the disposal state according to the present embodiment, the opening H is first formed, when disposing of the pad 30 in the state of not having an opening formed in advance; however, it is not limited thereto. Formation of the opening H with the perforation M (opening-forming means) as a starting point may be performed at any stage before fixing the portion U in the non-skin-side surface, which has been folded back toward the skin side to form the opening H, to the non-skin-side surface of the pad 30 that has been rolled up to the predetermined position (see FIG. 12B).

Regarding the fixing means (adhesive portion 3a), when used to maintain the rolled shape at the time of disposal, as in the pad 30, the fixing means is a fixing means that can fix the absorbent article (pad 30) to another article (such as underwear, another disposable diaper, or the like) during usage of the absorbent article (pad 30), and the fixing means that can also fix the absorbent article (pad 30) to the non-skin-side surface thereof. For example, when fixing the pad 30 to the skin-side surface of another disposable diaper, the fixing means in the non-skin-side surface of the pad 30 includes a male member of a hook-and-loop fastener, and the non-skin-side surface (back sheet 3) of the pad 30 is an exterior sheet of nonwoven fabric. Then, during usage, the pad 30 is fixed by engagement of the hook-and-loop fastener with the nonwoven fabric on the skin-side surface of the other disposable diaper, and when the pad 30 is disposed of, the rolled state of the pad 30 can be maintained by engaging the hook-and-loop fastener in the non-skin-side surface of the portion U corresponding to the opening H formed by the perforation M with the non-skin-side surface of the rolled pad 30.

Further, it is preferable that, when the pad 30 is viewed in the thickness direction, the area of a portion in which the opening-forming means (perforation M) and the fixing means (adhesive portion 3a) overlap each other is 1/2 or more of the area of the convex portion (portion U in FIG. 11B) and the like) that can be folded back toward the skin side. For example, when an adhesive such as a hot-melt adhesive is applied without a gap over the entire area of the adhesive portion 3a, the area of the adhesive portion 3a in the portion U is 100%. When an adhesive such as a hot-melt adhesive is applied in a predetermined pattern (such as a spiral pattern, a Ω-shaped pattern, a stripe pattern, or the like), it is preferable that the total area of the region where the adhesive is applied in the portion U is 1/2 or more of the area of the portion U. Accordingly, the pad 30 in the rolled state can be more firmly maintained by the portion U.

Regarding the perforation M (opening-forming means), as shown in FIG. 10, it is preferable that the perforation M is provided at a position extending across a center C-C of the pad 30 in the width direction when the pad 30 is viewed in the thickness direction. When fixing the rolled pad 30 by the adhesive portion 3a provided at a position overlapping the perforation M in the thickness direction, at the time of disposal of the pad 30, fixing it at a central portion thereof in the width direction allows the rolled shape of the pad 30 to be maintained more stably than when fixing it at an end portion thereof in the width direction.

Regarding the opening-forming means, the length WM of the perforation M (opening-forming means) in the width direction may be 1/2 or more of the maximum length W11 of the absorbent core 11 in the width direction (WM ≥ W11 × 1/2). Specifically, in FIG. 10, it refers to the length WM in the width direction from the end Mea end of the perforation M of the pad 30 located farthest on one side (left side) in the width direction to the end Meb thereof located farthest on the other side (right side) in the width direction. The larger the portion U folded back toward the skin side by the perforation M and the wider the area of the adhesive portion 3a in the portion U, the easier it becomes to maintain the rolled state of the pad 30. Thus, the length WM of the perforation M in the width direction being 1/2 or more of the length W11 of the absorbent core 11 in the width direction can more easily maintain the rolled state of the pad 30, as compared with the case where the length WM of the perforation M in the width direction is less than 1/2 of the maximum length W11 of the absorbent core 11 in the width direction.

As the opening-forming means, the length WM (opening-forming means) of the perforation M in the width direction may be 1/4 or more of the maximum length H11 of the absorbent core 11 in the front-back direction (WM ≥ H11 × 1/4). With the length WM of the perforation M in the width direction being 1/4 or more of the maximum length H11 of the absorbent core 11 in the front-back direction can more easily maintain the rolled state of the pad 30, as compared with the case where the length WM of the perforation M in the width direction is less than 1/4 of the maximum length H11 of the absorbent core 11 in the front-back direction.

When the perforation M is provided on the outer side in the front-back direction with respect to the end 11ea of the absorbent core 11 on one end in the front-back direction as in the pad 30, it is preferable that, in the width direction, the one end Mea of the perforation M located farthest on one side (for example, the left side in FIG. 10) is provided on the inner side (the other side, the right side in FIG. 10) with respect to the end 11sa of the absorbent core 11 located farthest on the one side in the width direction. Accordingly, it is possible to reduce the risk that the opening H becomes excessively large due to the perforation M, and to reduce the risk that the opening H formed by the perforation M reaches a side edge of the pad 30 on one side in the width direction and is no longer formed as a closed loop.

On the other hand, when the perforation M is provided on the outer side in the front-back direction with respect to the end 11ea on the one side (for example, the front side) of the absorbent core 11 in the front-back direction, the end Mea of the perforation M farthest on one side (for example, the left side in FIG. 10) in the width direction may be provided on the outer side (one side) with respect to the end 11sa of the absorbent core 11 farthest on the one side in the width direction. Accordingly, since the size of a portion pulled up toward the skin side when forming the opening H can be increased, the rolled pad 30 can be fixed over a wider area by the adhesive portion 3a in the non-skin-side surface of the opening H folded back toward the skin side, thereby making it easier to maintain the rolled state of the pad 30.

Further, the pad 30 includes leak-proof wall portions 6 in a pair on two sides in the width direction, respectively. As described above, each of the leak-proof wall portions 6 includes the leak-proof-wall elastic member 7 that can stretch and contract in the front-back direction, the rising portion 6b capable of rising toward the skin side, and the adhesion regions A1 and A2 that are the fixed portions 6a provided on the outer side in the front-back direction with respect to the rising portion 6b. The fixed portion 6a includes the first fixed portion 6a1 provided on the outer side in the width direction with respect to the rising portion 6b, and the second fixed portion 6a2 provided on the outer side in the front-back direction with respect to the rising portion 6b. When such leak-proof wall portions 6 are provided, it is preferable that, the perforation M includes a portion overlapping the second fixed portion 6a2 in the front-back direction. Further, in the width direction, it is preferable that the end Mea of the perforation M on the one side in the width direction is provided on the inner side with respect to the end ae2 on the one side in the width direction of the second fixed portion 6a2 on the one side in the width direction. Since the fixed portion 6a including the second fixed portion 6a2 (adhesion regions A1, A2) is a portion in which sheets constituting the leak-proof wall portion 6 are fixed by an adhesive such as a hot-melt adhesive or the like, it has higher stiffness than surrounding portions such as the rising portion 6b. Accordingly, with the perforation M being provided at a position in which it includes the portion overlapping the second fixed portion 6a2 in the front-back direction and the end Mea of the perforation M on the one side in the width direction being provided on the inner side (the other side) in the width direction with respect to the end ae2 of the second fixed portion 6a2 on the one side in the width direction, it is possible to reduce the risk that the opening H formed starting from the perforation M becomes excessively large and to reduce the risk that the opening H exceeds the end ae2 of the second fixed portion 6a2 on the one side in the width direction and reaches a side edge of the pad 30 on the one side in the width direction.

### <<< Modification of Third Embodiment >>>

Although the pad 30 according to the third embodiment has been described above, it is not limited thereto. The opening-forming means of the pad 30 in the third embodiment is a single substantially semicircular perforation M having a convex portion protruding toward a central portion in the front-back direction; however, it is not limited thereto. The shape, size, number, and arrangement of the opening-forming means can be set freely. Further, in the pad 30 of the third embodiment, the opening-forming means (perforations M) are provided on both of the front side and the back side in the front-back direction; however, it is not limited thereto, and the opening-forming means (perforation M) may be provided on either one of the front side or the back side of the pad 30.

Hereinabove, embodiments of the present invention have been described, however, the embodiments of the present invention described above are simply to facilitate understanding of the present disclosure and is/are not in any way to be construed as limiting the present invention. The present invention may variously be changed or altered without departing from its essential features and encompass equivalents thereof.

In the above embodiments, the pads 1, 20, and 30 taken out from a container material have been described; however, it is not limited thereto. For example, a container material may be an individual packaging sheet that individually wraps pad products such as sanitary napkins, panty liners, light incontinence products, light incontinence liners, urine-absorbing pads, and the like, and the pad products may be in a state removed from such an individual packaging sheet.

### REFERENCE SIGNS LIST

1, 20, 30 absorbent pad (pad, absorbent article)
2 top sheet,
3 back sheet,
3a adhesive portion (fixing means),
5 side sheet,
6 leak-proof wall portion,
6a fixed portion,
6b rising portion,
7 leak-proof wall elastic member,
8 leg elastic member (leg stretching/contracting member),
10 absorbent body,
11 absorbent core,
LG leg gather portion,
M perforation (opening forming means),
H opening

## Claims

1. An absorbent article having a front-back direction, a width direction, and a thickness direction orthogonal to each other in an unfolded state, the absorbent article comprising:
an absorbent core; and
an opening forming means,
in a state where the absorbent article has been taken out from a container material or a state where the absorbent article has been removed from the container material,
the absorbent article including neither a fastening tape nor a post-handling tape, the post-handling tape being used when the absorbent article is disposed of,
the opening forming means being configured to form an opening when the absorbent article is disposed of,
the opening being configured to allow insertion of at least a portion of the absorbent core when the absorbent article is disposed of, and
when viewed in the thickness direction, the opening-forming means being provided at a position not overlapping the absorbent core.

2. An absorbent article having a front-back direction, a width direction, and a thickness direction orthogonal to each other in an unfolded state, the absorbent article comprising:
an absorbent core; and
an opening forming means,
in a state where the absorbent article has been taken out from a container material or a state where the absorbent article has been removed from the container material,
the absorbent article not including a material having 60 g/m² or more,
the opening-forming means being configured to form an opening when the absorbent article is disposed of,
the opening being configured to allow insertion of at least a portion of the absorbent core when the absorbent article is disposed of,
when viewed in the thickness direction, the opening-forming means being provided at a position not overlapping the absorbent core.

3. An absorbent article having a front-back direction, a width direction, and a thickness direction orthogonal to each other in an unfolded state, the absorbent article comprising:
an absorbent core;
a fixing means; and
an opening forming means,
in a state where the absorbent article has been taken out from a container material or a state where the absorbent article has been removed from the container material,
the absorbent article not including a post-handling tape used when the absorbent article is disposed of,
the fixing means being located at a non-skin-side surface of the absorbent article, the fixing means being configured to fix the absorbent article to another article,
the opening forming means being configured to form an opening, when the absorbent article is disposed of, and
when viewed in the thickness direction,
the opening-forming means being provided at a position not overlapping the absorbent core, and
the opening-forming means and the fixing means having an overlapping portion.

4. The absorbent article according to any one of claims 1 to 3, wherein the opening-forming means is a perforation.

5. The absorbent article according to any one of claims 1 to 3, wherein at least one of a length of the opening-forming means in the front-back direction or a length thereof in the width direction is 1/2 or more of a maximum length of the absorbent core in the width direction.

6. The absorbent article according to any one of claims 1 to 3, wherein at least one of a length of the opening-forming means in the front-back direction or a length thereof in the width direction is 1/4 or more of a maximum length of the absorbent core in the front-back direction.

7. The absorbent article according to any one of claims 1 to 3, wherein in the front-back direction, the opening-forming means is provided on an outer side with respect to an end of the absorbent core on one side in the front-back direction.

8. The absorbent article according to claim 7, wherein in the front-back direction, the opening-forming means is further provided on an outer side with respect to an end of the absorbent core on an other side in the front-back direction as well.

9. The absorbent article according to claim 7, wherein on one side in the width direction, an end of the opening-forming means located farthest on one side is provided on an outer side with respect to an end of the absorbent core located farthest on the one side in the width direction.

10. The absorbent article according to claim 7, wherein in the width direction, an end of the opening-forming means located farthest on one side is provided on an inner side with respect to an end of the absorbent core located farthest on the one side.

11. The absorbent article according to claim 7, further comprising:
a pair of leak-proof wall portions on two sides in the width direction, wherein
the leak-proof wall portions each includes
a leak-proof wall elastic member that stretches and contracts in the front-back direction,
a rising portion capable of rising toward a skin side, and
a fixed portion not capable of rising,
the fixed portion including a first fixed portion and a second fixed portion,
the first fixed portion being provided on an outer side in the width direction with respect to the rising portion,
the second fixed portion being provided on an outer side in the front-back direction with respect to the rising portion,
in the front-back direction, the opening-forming means and the second fixed portion have an overlapping portion, and
in the width direction, an end of the opening-forming means on one side in the width direction is provided on an inner side with respect to an end of the second fixed portion on the one side in the width direction, the second fixed portion being provided on the one side in the width direction.

12. The absorbent article according to claim 1 or 2, wherein the opening-forming means is provided on an outer side in the width direction with respect to a side end of the absorbent core.

13. The absorbent article according to claim 12, further comprising:
a pair of leak-proof wall portions on two sides in the width direction, wherein
the leak-proof wall portions each includes
a leak-proof wall elastic member that stretches and contracts in the front-back direction,
a rising portion capable of rising toward a skin side, and
a fixed portion not capable of rising,
he fixed portion including a first fixed portion and a second fixed portion,
the first fixed portion being provided on an outer side in the width direction with respect to the rising portion,
the second fixed portion being provided on an outer side in the front-back direction with respect to the rising portion, and
on one side with respect to a center in the width direction, the opening-forming means is provided on the outer side in the width direction with respect to an end of the first fixed portion on the one side in the width direction.

14. The absorbent article according to claim 13, further comprising:
leg gather portions on two sides in the width direction, the leg gather portions being arranged around legs of a wearer during usage, wherein
the leg gather portions include leg stretching/contracting members extending along the front-back direction, and
on the one side with respect to the center in the width direction, the opening-forming means is provided on an inner side in the width direction with respect to the leg stretching/contracting members.

15. The absorbent article according to claim 12, further comprising:
leg gather portions on two sides in the width direction, the leg gather portions being arranged around legs of a wearer during usage, wherein
the leg gather portions include leg stretching/contracting members extending along the front-back direction, and
a length of the opening-forming means in the front-back direction is shorter than a length of a portion capable of stretching and contracting of the leg stretching/contracting members.

16. The absorbent article according to claim 12, further comprising:
leg gather portions on two sides in the width direction, the leg gather portions being arranged around legs of a wearer during usage, wherein
the leg gather portions include leg stretching/contracting members extending along the front-back direction, and
a length of the opening-forming means in the front-back direction is longer than a length of a portion capable of stretching and contracting of the leg stretching/contracting members.

17. The absorbent article according to claim 12, further comprising:
a fixing means at a non-skin-side surface of the absorbent article, the fixing means being configured to fix the absorbent article to another article, wherein
on one side in the front-back direction, at least a portion of the fixing means is provided on one side in the front-back direction with respect to the opening-forming means.

18. The absorbent article according to claim 1 or 2, further comprising:
a fixing means at a non-skin-side surface of the absorbent article, the fixing means being configured to fix the absorbent article to another article, wherein
when viewed in the thickness direction, the opening-forming means and the fixing means have an overlapping portion.

19. The absorbent article according to claim 1 or 2, further comprising:
a fixing means at a non-skin-side surface of the absorbent article, the fixing means being configured to fix the absorbent article to another article, wherein
when viewed in the thickness direction, the opening-forming means and the fixing means do not have an overlapping portion.

20. The absorbent article according to claim 3, wherein
the opening-forming means includes a convex portion, and
the convex portion is configured to be capable of being folded back toward a skin side when forming the opening.

21. The absorbent article according to claim 20, wherein the convex portion has a convex shape that protrudes toward a center in the front-back direction.

22. The absorbent article according to claim 20, wherein when viewed in the thickness direction, an area of an overlapping portion between the opening-forming means and the fixing means is 1/2 or more of an area of the convex portion capable of being folded back toward the skin side.

23. The absorbent article according to claim 20, wherein when viewed in the thickness direction, the opening-forming means is provided at a position extending across a center of the absorbent article in the width direction.
